(19) Europäisches Patentamt
European Patent Office
Office européen des brevets

(11) EP 2 313 194 B1

(12) FASCICULE DE BREVET EUROPEEN

(45) Date de publication et mention de la délivrance du brevet:
**19.09.2012 Bulletin 2012/38**

(21) Numéro de dépôt: **09784284.3**

(22) Date de dépôt: **21.07.2009**

(51) Int Cl.:
***B01J 29/035*** (2006.01) ***B01J 29/04*** (2006.01)
***C01B 39/48*** (2006.01) ***B01J 29/70*** (2006.01)
***C07C 1/24*** (2006.01) ***C07C 2/08*** (2006.01)
***C07C 2/66*** (2006.01) ***C07C 6/12*** (2006.01)
***C11C 3/06*** (2006.01) ***C01B 39/46*** (2006.01)
***C07C 11/02*** (2006.01) ***C10G 45/12*** (2006.01)
***C10G 50/00*** (2006.01) ***C11C 3/00*** (2006.01)

(86) Numéro de dépôt international:
**PCT/FR2009/000898**

(87) Numéro de publication internationale:
**WO 2010/015732 (11.02.2010 Gazette 2010/06)**

# (54) CATALYSEUR COMPRENANT AU MOINS UNE ZEOLITHE IZM-2 ET SON UTILISATION POUR LA TRANSFORMATION DE CHARGES HYDROCARBONEES

KATALYSATOR MIT MINDESTENS EINEM IZM-2-ZEOLITH UND SEINE VERWENDUNG ZUR UMWANDLUNG VON ROHSTOFFEN AUF KOHLENWASSERSTOFFBASIS

CATALYST COMPRISING AT LEAST ONE IZM-2 ZEOLITE AND USE THEREOF FOR THE CONVERSION OF HYDROCARBON-BASED FEEDSTOCKS

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **08.08.2008 FR 0804561**

(43) Date de publication de la demande:
**27.04.2011 Bulletin 2011/17**

(73) Titulaire: **IFP Energies nouvelles**
**92852 Rueil-Malmaison Cedex (FR)**

(72) Inventeurs:
- **CABIAC, Amandine**
  **F-69007 Lyon (FR)**
- **CADRAN, Nicolas**
  **F-69600Oullins (FR)**
- **GUILLON, Emmanuelle**
  **F-69390 Vernaison (FR)**
- **LECOQ, Vincent**
  **F-69530 Brignais (FR)**
- **MAURY, Sylvie**
  **F-69390 Charly (FR)**

(56) Documents cités:
**EP-A- 1 702 888** **EP-A- 1 953 118**
**WO-A-2009/004131** **WO-A1-2009/144414**
**US-A- 4 539 193**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 313 194 B1

## Description

La présente invention se rapporte au domaine des catalyseurs zéolithiques et à leur utilisation dans différents procédés de transformation de charges hydrocarbonées. Plus précisément, la présente invention se rapporte à un catalyseur comprenant au moins une zéolithe IZM-2 et au moins une matrice.

### Etat de la technique antérieure

Les matériaux microporeux cristallisés, tel que les zéolithes ou les silicoaluminophosphates, sont des solides très utilisés dans l'industrie pétrolière en tant que catalyseur, support de catalyseur, adsorbant ou agent de séparation. Bien que de nombreuses structures cristallines microporeuses aient été découvertes, l'industrie du raffinage et de la pétrochimie est toujours à la recherche de nouvelles structures zéolithiques qui présentent des propriétés particulières pour des applications comme la purification ou la séparation des gaz, la conversion d'espèces carbonées ou autres. Les propriétés d'un catalyseur zéolithique dépendent fortement de la structure poreuse de la zéolithe qu'il contient, de sa stabilité et de son acidité.
Le document EP19531-18 A décrit un catalyseur comprenant une zeolithe IZM-1, ladite zéolithe présentant une composition chimique, exprimée sur une base anhydre, en termes de moles d'oxydes, par la formule générale suivante : $XO_2$ : $aY_2O_3$ : $bM_nO$.

### Résumé et intérêt de l'invention

La présente invention porte sur un catalyseur comprenant au moins une zéolithe IZM-2 et au moins une matrice, ladite zéolithe présentant un diagramme de diffraction des rayons X incluant au moins les raies inscrites dans le tableau 1 et présentant une composition chimique, exprimée sur une base anhydre, en termes de moles d'oxydes, par la formule générale suivante : $XO_2$ : $aY_2O_3$ : $bM_nO$ dans laquelle X représente au moins un élément tétravalent, Y représente au moins un élément trivalent et M est au moins un métal alcalin et/ou un métal alcalino-terreux, a et b représentant respectivement le nombre de moles de $Y_2O_3$ et $M_nO$ et a est compris entre 0,001 et 0,5, b est compris entre 0 et 1 et n est compris entre 1 et 2.
Ledit catalyseur selon l'invention est avantageusement utilisé pour la mise en oeuvre de différents procédés de transformation de charges hydrocarbonées. En particulier, ledit catalyseur selon l'invention conduit à des performances catalytiques intéressantes lorsqu'il est utilisé dans la transformation d'hydrocarbures, d'alcools ou encore de triglycérides.

### Description de l'invention

La présente invention porte sur un catalyseur comprenant au moins une zéolithe IZM-2 et au moins une matrice, ladite zéolithe présentant un diagramme de diffraction des rayons X incluant au moins les raies inscrites dans le tableau 1 ci-dessous :

Tableau 1 : Valeurs moyennes des $d_{hkl}$ et intensités relatives mesurées sur un diagramme de diffraction de rayons X de la zéolithe IZM-2 calcinée

| 2 thêta (°) | $d_{hkl}$ (Å) | Irel | 2 thêta (°) | $d_{hkl}$ (Å) | Irel |
|---|---|---|---|---|---|
| 5,07 | 17,43 | ff | 19,01 | 4,66 | ff |
| 7,36 | 12,01 | FF | 19,52 | 4,54 | ff |
| 7,67 | 11,52 | FF | 21,29 | 4,17 | m |
| 8,78 | 10,07 | F | 22,44 | 3,96 | f |
| 10,02 | 8,82 | ff | 23,10 | 3,85 | mf |
| 12,13 | 7,29 | ff | 23,57 | 3,77 | f |
| 14,76 | 6,00 | ff | 24,65 | 3,61 | ff |
| 15,31 | 5,78 | ff | 26,78 | 3,33 | f |
| 15,62 | 5,67 | ff | 29,33 | 3,04 | ff |
| 16,03 | 5,52 | ff | 33,06 | 2,71 | ff |

(suite)

| 2 thêta (°) | $d_{hkl}$ (Å) | Irel | 2 thêta (°) | $d_{hkl}$ (Å) | Irel |
|---|---|---|---|---|---|
| 17,60 | 5,03 | ff | 36,82 | 2,44 | ff |
| 18,22 | 4,87 | ff | 44,54 | 2,03 | ff |

où FF = très fort ; F = fort ; m = moyen ; mf = moyen faible ; f = faible ; ff = très faible et présentant une composition chimique, exprimée sur une base anhydre, en termes de moles d'oxydes, par la formule générale suivante : $XO_2$ : $aY_2O_3$ : $bM_nO$ dans laquelle X représente au moins un élément tétravalent, Y représente au moins un élément trivalent et M est au moins un métal alcalin et/ou un métal alcalino-terreux, a et b représentant respectivement le nombre de moles de $Y_2O_3$ et $M_nO$ et a est compris entre 0,001 et 0,5, b est compris entre 0 et 1 et n est compris entre 1 et 2.

Le diagramme de diffraction dont les données figurent dans le tableau 1 est obtenu par analyse radiocristallographique au moyen d'un diffractomètre en utilisant la méthode classique des poudres avec le rayonnement $K\alpha_1$ du cuivre ($\lambda$ = 1,5406Å). A partir de la position des pics de diffraction représentée par l'angle 2θ, on calcule, par la relation de Bragg, les équidistances réticulaires $d_{hkl}$ caractéristiques de l'échantillon. L'erreur de mesure $\Delta(d_{hkl})$ sur $d_{hkl}$ est calculée grâce à la relation de Bragg en fonction de l'erreur absolue $\Delta(2\theta)$ affectée à la mesure de 2θ. Une erreur absolue $\Delta(2\theta)$ égale à ± 0,02° est communément admise. L'intensité relative $I_{rel}$ affectée à chaque valeur de $d_{hkl}$ est mesurée d'après la hauteur du pic de diffraction correspondant. Le diagramme de diffraction des rayons X de la zéolithe IZM-2 présente dans le catalyseur selon l'invention comporte au moins les raies aux valeurs de $d_{hkl}$ données dans le tableau 1. Dans la colonne des $d_{hkl}$, on a indiqué les valeurs moyennes des distances inter-réticulaires en Angströms (Å). Chacune de ces valeurs doit être affectée de l'erreur de mesure $\Delta(d_{hkl})$ comprise entre ± 0,6Å et ± 0,01Å.

La zéolithe IZM-2 présente dans le catalyseur selon l'invention présente une composition chimique exprimée sur une base anhydre, en termes de moles d'oxydes, définie par la formule générale suivante : $XO_2$ : $aY_2O_3$ : $bM_nO$, dans laquelle X représente au moins un élément tétravalent, Y représente au moins un élément trivalent et M est au moins un métal alcalin et/ou un métal alcalino-terreux. Dans ladite formule donnée ci-dessus, a représente le nombre de moles de $Y_2O_3$ et a est compris entre 0,001 et 0,5, de préférence entre 0,001 et 0,05 et de manière très préférée entre 0,001 et 0,02 et b représente le nombre de moles de $M_nO$ et est compris entre 0 et 1, de préférence entre 0 et 0,5 et de manière encore plus préférée entre 0,005 et 0,5. n est compris entre 1 et 2, de préférence, n est égal à 1 ou n est égal à 2.

Conformément à l'invention, X est préférentiellement choisi parmi le silicium, le germanium, le titane et le mélange d'au moins deux de ces éléments tétravalents, très préférentiellement X est le silicium et Y est préférentiellement choisi parmi l'aluminium, le bore, le fer, l'indium et le gallium, très préférentiellement Y est l'aluminium. Dans la zéolithe IZM-2 présente dans le catalyseur selon l'invention, X est préférentiellement le silicium et Y est préférentiellement l'aluminium. M est préférentiellement choisi parmi le lithium, le sodium, le potassium, le césium, le rubidium, le calcium, le magnésium et le baryum et le mélange d'au moins deux de ces métaux et très préférentiellement M est le sodium et/ou le césium.

La zéolithe IZM-2 présente dans le catalyseur selon l'invention et contenant des atomes X et Y tels que définis ci-dessus, de préférence des atomes d'aluminium et des atomes de silicium, présente un rapport atomique X/Y global, de préférence un rapport atomique Si/Al global, compris entre 5 et 100, de préférence compris entre 10 et 50 et de manière très préférée compris entre 10 et 35. La zéolithe IZM-2 présente dans le catalyseur selon l'invention peut également être désaluminée. La zéolithe IZM-2 présente dans le catalyseur selon l'invention se présente très avantageusement sous sa forme protonée (forme hydrogène $H^+$) dans laquelle la proportion en cation autre que $H^+$ est inférieure à 30% du nombre total de cations, de préférence inférieure à 20% et de manière très préférée inférieure à 5% par rapport au nombre total de cations sur la zéolithe. Conformément à l'invention, lorsque la zéolithe IZM-2 se trouve sous sa forme protonée, le coefficient b est nul dans la formule $XO_2$ : $aY_2O_3$ : $bM_nO$ donnée ci-dessus. Néanmoins, pour des applications telles que la fabrication d'esters alcooliques à partir de triglycérides et d'alcools comme décrit ci-après, la zéolithe IZM-2 présente dans le catalyseur selon l'invention se présente sous forme basique, la proportion en cations alcalins et/ou alcalino-terreux autre que $H^+$ étant très supérieure à 30% du nombre total de cations, de préférence supérieure à 70% et de manière très préférée supérieure à 80% du nombre total de cations. Lorsque ladite zéolithe IZM-2 présente dans le catalyseur selon l'invention est sous forme basique, elle est préférentiellement échangée avec le césium.

La matrice présente dans le catalyseur selon l'invention est une matrice minérale poreuse, généralement amorphe. Elle est choisie parmi les éléments du groupe formé par les alumines, les silices, la magnésie, les silices-alumines amorphes, les argiles naturelles (kaolin, bentonite, sepiolite, attapulgite), l'oxyde de titane, l'oxyde de bore, la zircone, les phosphates d'aluminium, les phosphates de titane, les phosphates de zirconium, le charbon et leurs mélanges. On préfère utiliser une matrice contenant de l'alumine, particulièrement sous toutes ses formes connues de l'Homme du métier et de manière encore plus préférée l'alumine gamma. On peut aussi avantageusement utiliser des mélanges d'alumine et de silice, des mélanges d'alumine et de silice-alumine.

Le catalyseur selon l'invention comprend avantageusement au moins un métal additionnel choisi parmi les métaux des groupes IIIB, IVA et IVB : ledit catalyseur comprend un métal choisi parmi les métaux des groupes IIIB, IVA et IVB ou plusieurs métaux en mélange choisis parmi les métaux des groupes IIIB, IVA et IVB, par exemple un métal du groupe IIIB et un métal du groupe IVB. Parmi les métaux du groupe IIIB, le lanthane et le cérium sont préférés. Parmi les métaux du groupe IVA, l'étain et le germanium sont préférés. Parmi les métaux du groupe IVB, le titane et le zirconium sont préférés.

Le catalyseur selon l'invention est dépourvu de toute phase sulfure.

Ledit catalyseur selon l'invention contient plus particulièrement :

- de 4 à 60% poids d'au moins ladite zéolithe IZM-2,
- de 40 à 96% poids d'au moins une matrice,
- éventuellement de 0,01 à 2%, de préférence de 0,05 à 1% poids d'au moins un métal additionnel choisi parmi les métaux des groupes IIIB, IVA et IVB.

Le catalyseur selon l'invention se présente préférentiellement sous forme de billes ou d'extrudés. Il présente des propriétés mécaniques telles que la valeur de l'écrasement en lit, déterminé selon la méthode Shell (SMS 1471-74) est de préférence supérieure à 0,7 MPa.

La présente invention a également pour objet la préparation du catalyseur selon l'invention. La préparation du catalyseur selon l'invention commence d'abord par la préparation de la zéolithe IZM-2.

Ladite zéolithe IZM-2 présente dans le catalyseur selon l'invention est préparée selon un procédé dans lequel on fait réagir un mélange aqueux comportant au moins une source d'au moins un oxyde $XO_2$, au moins une source d'au moins un oxyde $Y_2O_3$, au moins une source d'au moins un métal alcalin et/ou alcalino-terreux, au moins une espèce organique R comportant deux atomes d'azote quaternaires, le mélange présentant préférentiellement la composition molaire suivante :

| | |
|---|---|
| $XO_2/N_2O_3$ | : au moins 2, de préférence au moins 20, de manière plus préférée de 55 à 600, |
| $H_2O/XO_2$ | : 1 à 100, de préférence de 10 à 70, |
| $R/XO_2$ | : 0,02 à 2, de préférence de 0,05 à 0,5, |
| $M_nO/XO_2$ | : 0,001 à 1, de préférence de 0,005 et 0,5, |

où X est un ou plusieurs élément(s) tétravalent(s) choisi(s) dans le groupe formé par les éléments suivants : silicium, germanium, titane, de préférence le silicium, où Y est un ou plusieurs élément(s) trivalent(s) choisi(s) dans le groupe formé par les éléments suivants : aluminium, fer, bore, indium et gallium, de préférence l'aluminium et où M est un ou plusieurs metal(aux) alcalin(s) et/ou alcalino-terreux choisi(s) parmi le lithium, le sodium, le potassium, le rubidium, le césium, le calcium, le magnésium, le baryum et le mélange d'au moins deux de ces métaux de préférence le sodium.

R est une espèce organique ayant deux atomes d'azote quaternaires jouant le rôle de structurant organique. Préférentiellement, R est le composé azoté 1,6-bis(méthylpiperidinium)hexane dont la formule développée est donnée ci-dessous.

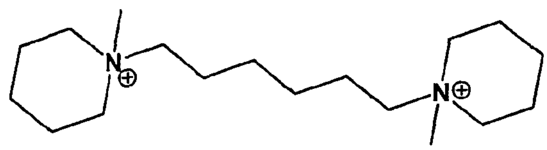

Les anions associés aux cations ammoniums quaternaires présents dans l'espèce organique structurante pour la synthèse de la zéolithe IZM-2 présente dans le catalyseur selon l'invention sont choisis parmi l'anion acétate, l'anion sulfate, l'anion carboxylate, l'anion tétrafluoroborate, les anions halogénures tels que le fluorure, le chlorure, le bromure, l'iodure, l'anion hydroxyde et une combinaison de plusieurs d'entre eux. De manière préférée, les anions associés aux cations ammoniums quaternaires présents dans l'espèce structurante pour la synthèse de la zéolithe IZM-2 sont choisis parmi l'anion hydroxyde et l'anion bromure. Ladite espèce organique azotée utilisée comme agent structurant de la zéolithe IZM-2 est synthétisée par toute méthode connue de l'Homme du métier. Pour la synthèse du dibromure de 1,6-bis(méthylpiperidinium)hexane, on procède au mélange d'une mole de 1,6-dibromohexane et d'au moins 2 moles de N-méthylpiperidine dans l'éthanol. Généralement, le mélange est porté à reflux pendant une durée comprise entre 3 et 10 heures. Après filtration, précipitation au moyen d'un solvant éthéré tel que le diéthyéther puis recristallisation dans un mélange éthanol/éther, on obtient du dibromure de 1,6-bis(méthylpiperidinium)hexane. Le dihydroxyde de 1,6-bis (méthylpiperidinium)hexane est préférentiellement obtenu par traitement à température ambiante d'une solution aqueuse

de dibromure de 1,6-bis(méthylpiperidinium)hexane par de l'oxyde d'argent $Ag_2O$.

La source de l'élément X, employée pour la mise en oeuvre du procédé de préparation de la zéolithe IZM-2, peut être tout composé comprenant l'élément X et pouvant libérer cet élément en solution aqueuse sous forme réactive. Avantageusement, lorsque l'élément X est le silicium, la source de silice peut être l'une quelconque de celles couramment utilisées dans la synthèse des zéolithes, par exemple de la silice solide en poudre, de l'acide silicique, de la silice colloïdale, de la silice dissoute ou du tétraéthoxysilane (TEOS). Parmi les silices en poudre, on peut utiliser les silices précipitées, notamment celles obtenues par précipitation à partir d'une solution de silicate de métal alcalin, telle que des silices aérosiles, des silices pyrogénées, par exemple du "CAB-O-SIL" et des gels de silice. On peut utiliser des silices colloïdales présentant différentes tailles de particules, par exemple de diamètre équivalent moyen compris entre 10 et 15 nm ou entre 40 et 50 nm telles que celles commercialisées sous les marques déposées telle que "LUDOX". De manière préférée, la source de silicium est le LUDOX AS-40.

La source de l'élément Y employée pour la mise en oeuvre du procédé de préparation de la zéolithe IZM-2 peut être tout composé comprenant l'élément Y et pouvant libérer cet élément en solution aqueuse sous forme réactive. Dans le cas préféré où Y est l'aluminium, la source d'alumine est de préférence de l'aluminate de sodium, ou un sel d'aluminium, par exemple du chlorure, du nitrate, de l'hydroxyde ou du sulfate, un alkoxyde d'aluminium ou de l'alumine proprement dite, de préférence sous forme hydratée ou hydratable, comme par exemple de l'alumine colloïdale, de la pseudoboehmite, de l'alumine gamma ou du trihydrate alpha ou bêta. On peut également utiliser des mélanges des sources citées ci-dessus.

Pour la source de métal M alcalin et/ou alcalino-terreux, on utilise avantageusement un halogénure ou un hydroxyde dudit métal M, de préférence un hydroxyde dudit métal M.

Pour la mise en oeuvre du procédé de préparation de la zéolithe IZM-2, il est préféré que le mélange aqueux, comportant au moins une source d'au moins un oxyde $XO_2$, au moins une source d'au moins un oxyde $Y_2O_3$, au moins une source d'au moins un métal alcalin et/ou alcalino-terreux, au moins une espèce organique R ayant deux atomes d'azote quaternaires, comporte également au moins une source d'ions hydroxydes. Ladite source d'ions hydroxydes provient avantageusement de l'espèce organique structurante R lorsqu'elle se trouve sous sa forme hydroxyde, à savoir le dihydroxyde de 1,6-bis(méthylpiperidinium)hexane, ou encore de la source de métal M alcalin et/ou alcalino-terreux lorsqu'il se trouve sous forme hydroxyde, par exemple l'hydroxyde de sodium. Aussi, selon un mode de réalisation préféré du procédé de préparation de la zéolithe IZM-2 présente dans le catalyseur selon l'invention, on fait réagir un mélange aqueux comportant un oxyde de silicium, de l'alumine, du dibromure de 1,6-bis(méthylpiperidinium)hexane et de l'hydroxyde de sodium.

Le procédé de préparation de la zéolithe IZM-2 présente dans le catalyseur selon l'invention consiste à préparer un mélange réactionnel aqueux appelé gel et renfermant au moins une source d'au moins un oxyde $XO_2$, au moins une source d'au moins un oxyde $Y_2O_3$, au moins une espèce organique R, au moins une source d'au moins un métal alcalin et/ou alcalino-terreux. Les quantités desdits réactifs sont ajustées de manière à conférer à ce gel une composition permettant sa cristallisation en zéolithe IZM-2 sous sa forme brute de synthèse de formule générale (I) $XO_2 : aY_2O_3 : bM_nO : cR : dH_2O$, où a, b, et n répondent aux critères définis plus haut, c représente le nombre de moles de R et est compris entre 0,005 et 2, de préférence entre 0,01 et 0,5 et d représente le nombre de moles de $H_2O$ et est compris entre 0,005 et 2 et de préférence entre 0,01 et 1. Puis le gel est soumis à un traitement hydrothermal jusqu'à ce que la zéolithe IZM-2 se forme. Le gel est avantageusement mis sous conditions hydrothermales sous une pression de réaction autogène, éventuellement en ajoutant du gaz, par exemple de l'azote, à une température comprise entre 120°C et 200°C, de préférence entre 140°C et 180°C, et de manière encore plus préférée entre 160 et 175°C jusqu'à la formation des cristaux de zéolithe IZM-2 sous sa forme brute de synthèse. La durée nécessaire pour obtenir la cristallisation varie généralement entre 1 heure et plusieurs mois en fonction de la composition des réactifs dans le gel, de l'agitation et de la température de réaction. De préférence la durée de cristallisation varie entre 2 heures et 21 jours. La mise en réaction s'effectue généralement sous agitation ou en absence d'agitation, de préférence en présence d'agitation.

Il peut être avantageux d'additionner des germes au mélange réactionnel afin de réduire le temps nécessaire à la formation des cristaux et/ou la durée totale de cristallisation. Il peut également être avantageux d'utiliser des germes afin de favoriser la formation de la zéolithe IZM-2 au détriment d'impuretés. De tels germes comprennent des solides cristallisés, notamment des cristaux de zéolithe IZM-2. Les germes cristallins sont généralement ajoutés dans une proportion comprise entre 0,01 et 10 % de la masse de l'oxyde $XO_2$ utilisée dans le mélange réactionnel.

A l'issue de l'étape de traitement hydrothermal conduisant à la cristallisation de la zéolithe IZM-2, la phase solide est filtrée et lavée, on obtient ainsi la zéolithe IZM-2 sous sa forme brute de synthèse laquelle est séchée puis calcinée pour obtenir la zéolithe sous forme calcinée. L'étape de calcination s'effectue avantageusement par une ou plusieurs étapes de chauffage réalisée à une température comprise entre 100 et 1000°C, de préférence comprise entre 400 et 650°C, pour une durée comprise entre quelques heures et plusieurs jours, de préférence comprise entre 3 heures et 48 heures. De manière préférée, la calcination s'effectue en deux étapes de chauffage consécutives. A l'issue de ladite étape de calcination, la zéolithe IZM-2 obtenue est celle présentant le diagramme de diffraction de rayons X incluant au moins les raies inscrites dans le tableau 1. Elle est dépourvue d'eau ainsi que de l'espèce organique R

présentes dans la zéolithe IZM-2 sous sa forme brute de synthèse.

Selon un premier mode de réalisation du procédé de préparation du catalyseur selon l'invention, ledit catalyseur est préparé selon un procédé comprenant au moins les étapes :

a) le traitement de la zéolithe IZM-2 sous sa forme calcinée par au moins un échange ionique de manière à obtenir ladite zéolithe sous forme hydrogène ;
b) la mise en forme de ladite zéolithe sous forme hydrogène avec au moins une matrice minérale poreuse ;
c) la calcination du solide issu de ladite étape b).

Selon l'étape a) dudit premier mode de réalisation, la zéolithe IZM-2 calcinée, préparée conformément au procédé décrit ci-dessus, est soumise à un ou plusieurs échange(s) ionique(s) par une solution contenant au moins un sel d'ammonium, par exemple le nitrate d'ammonium $NH_4NO_3$, de manière à éliminer au moins en partie, de préférence pratiquement totalement, le(s) cation(s) alcalin/alcalino-terreux présent(s) dans la zéolithe IZM-2 calcinée. Une étape de calcination sous flux d'air sec, à une température généralement comprise entre 400 et 500°C, a ensuite pour but de générer la formation des protons dans la zéolithe par désorption d'ammoniaque conduisant ainsi à la forme hydrogène de la zéolithe. Ladite zéolithe est alors une zéolithe acide contenant entre 70 et 100%, de préférence entre 80 et 100% et de manière très préférée entre 85 et 100% de cations de compensation de forme protonique $H^+$, le reste des cations étant choisi parmi les métaux des groupes IA et IIA de la classification périodique des éléments et plus particulièrement ledit cation est choisi parmi les cations $Na^+$, $Li^+$, $K^+$, $Rb^+$, $Cs^+$, $Ba^{2+}$, $Mg^{2+}$, et $Ca^{2+}$.

Selon l'étape b) dudit premier mode de réalisation du procédé de préparation du catalyseur selon l'invention, la zéolithe IZM-2 sous forme hydrogène est mise en forme par toute technique connue de l'Homme du métier. Elle peut en particulier être mélangée avec au moins une matrice minérale poreuse, généralement amorphe, par exemple avec une poudre humide de gel d'alumine. Le mélange est ensuite mis en forme par exemple par extrusion au travers d'une filière. La mise en forme peut être réalisée avec d'autres matrice que l'alumine, telles que par exemple la magnésie, les silices-alumines amorphes, les argiles naturelles (kaolin, bentonite, sepiolite, attapulgite), les silices, l'oxyde de titane, l'oxyde de bore, la zircone, les phosphates d'aluminium, les phosphates de titane, les phosphates de zirconium, le charbon et leurs mélanges. On préfère utiliser des matrices contenant de l'alumine sous toutes ses formes connues de l'Homme du métier et de manière encore plus préférée l'alumine gamma. On peut aussi avantageusement utiliser des mélanges d'alumine et de silice, des mélanges d'alumine et de silice-alumine. D'autres techniques que l'extrusion telles que le pastillage et la dragéification peuvent être utilisées. Les conditions de mise en forme de la zéolithe IZM-2, le choix de la matrice, éventuellement le broyage préalable de la zéolithe, le procédé de peptisation, l'ajout d'agents porogènes, le temps de malaxage, la pression d'extrusion si le catalyseur est mis sous forme d'extrudés, la vitesse et le temps de séchage, sont déterminés, pour chaque matrice, selon les règles bien connues de l'homme de métier, de manière à obtenir un catalyseur de préférence sous forme d'extrudés ou de billes.

Selon ladite étape c) dudit premier mode de réalisation du procédé de préparation du catalyseur selon l'invention, le solide mis en forme issu de ladite étape b) est soumis à une étape de calcination réalisée à une température comprise entre 250 et 600°C de préférence entre 400 et 550°C. De manière préférée, ladite étape de calcination est précédée d'une étape de séchage réalisée à une température comprise entre 40 et 250°C, de préférence entre 80 et 200°C.

Selon une variante dudit premier mode de réalisation du procédé de préparation, les protons présents dans la zéolithe IZM-2 sous forme hydrogène issue de ladite étape a) sont échangés au moins en partie, préférentiellement totalement, au cours d'une étape a') par des cations appartenant à la famille des métaux alcalins et alcalino-terreux. En particulier, les protons présents dans ladite zéolithe IZM-2 sous forme hydrogène issue de ladite étape a) sont échangés par $Li^+$, $Na^+$, $K^+$, $Rb^+$, $Cs^+$, $Mg^{2+}$, $Ca^{2+}$ ou $Ba^{2+}$, préférentiellement par $Na^+$ ou $Cs^+$. Ladite étape a') qui précède ladite étape b) peut être effectuée par toute technique connue de l'Homme du métier et en particulier par imprégnation par excès ou par échange ionique selon les méthodes bien connues de l'Homme du métier, de préférence par échange(s) ionique(s). La zéolithe IZM-2 obtenue à l'issue de ladite a') est alors basique : les cations qu'elle contient sont majoritairement des cations choisis parmi les cations $Li^+$, $Na^+$, $K^+$, $Rb^+$, $Cs^+$, $Mg^{2+}$, $Ca^{2+}$ et $Ba^{2+}$, préférentiellement parmi les cations $Na^+$ et $Cs^+$. Ladite zéolithe IZM-2 sous forme basique peut contenir différents cations choisis parmi $Li^+$, $Na^+$, $K^+$, $Rb^+$, $Cs^+$, $Mg^{2+}$, $Ca^{2+}$ et $Ba^{2+}$, en particulier elle peut contenir des cations $Cs^+$ et $Na^+$. Elle peut également contenir un ou plusieurs cations choisi(s) parmi les cations $Li^+$, $Na^+$, $K^+$, $Rb^+$, $Cs^+$, $Mg^{2+}$, $Ca^{2+}$ et $Ba^{2+}$ et des protons lesquels sont présents dans une proportion inférieure à 30% du nombre total de cations présents dans ladite zéolithe IZM-2 sous forme basique. Ladite étape a') est mise en oeuvre en mettant en contact ladite zéolithe IZM-2 sous forme acide issue de ladite étape a) avec une solution contenant au moins un précurseur du(es) métal(ux) appartenant à la famille des métaux alcalins et alcalino-terreux, préférentiellement au moins un précurseur du(es) métal(ux) choisi(s) parmi Li, Na, K, Rb, Cs, Mg, Ca et Ba, et très préférentiellement au moins un précurseur du sodium ou du césium. Parmi les précurseurs employés pour la mise en oeuvre de ladite étape a'), on peut citer les oxydes métalliques et leurs mélanges en toute proportion ainsi que des sels des métaux alcalins et alcalino-terreux, notamment des sels d'halogénures, de sulfates, de nitrates, de phosphates, de carbonates, d'oxalates, d'hydroxydes, d'acétates, d'alcoolates, de perchlorates, de carboxylates ou d'acétylacétonates. Lorsque les protons présents dans ladite zéolithe IZM-2 sous forme acide issue de ladite étape a) sont échangés par des cations $Cs^+$, on utilise avantageusement de l'acétate de césium ou du nitrate de césium. Ces précurseurs peuvent être sous forme de poudre ou mis en forme, soluble ou insoluble dans le milieu réactionnel. Conformément à ladite variante dudit premier mode de réalisation, l'étape a') est suivie de la calcination de la zéolithe IZM-2 échangée sous forme basique à une température comprise entre 250 et 600°C avant de procéder à la mise en oeuvre de ladite étape b).

Conformément audit premier mode de réalisation dudit procédé de préparation du catalyseur selon l'invention, la zéolithe IZM-2 sous forme acide issue de ladite étape a) ou la zéolithe IZM-2 sous forme basique issue de ladite étape a') lorsque la variante est mise en oeuvre est avantageusement soumise à un ou plusieurs traitement(s) connu(s) de l'Homme du métier visant à stabiliser, désaluminer ou passiver ladite zéolithe.

Selon un deuxième mode de réalisation du procédé de préparation du catalyseur selon l'invention, ledit catalyseur est préparé selon un procédé comprenant au moins les étapes :

d) la mise en forme de ladite zéolithe IZM-2 avec au moins une matrice minérale poreuse ;
e) la calcination du solide issu de ladite étape d) ;
f) le traitement du solide issu de ladite étape e) par au moins un échange ionique de manière à obtenir ladite zéolithe sous une forme telle que la valeur prise par b dans la formule $XO_2$ :
$aY_2O_3$ $bM_nO$ soit nulle ou comprise entre 0,005 et 0,5, a et n ayant les mêmes valeurs que celles données plus haut dans la présente description, X, Y et M ayant été également définis plus haut dans la présente description ;
g) la calcination du solide issu de ladite étape f).

Conformément à l'étape d) dudit deuxième mode de réalisation du procédé de préparation du catalyseur selon l'invention et selon une première variante, on utilise pour ladite étape de mise en forme une zéolithe IZM-2 sous sa forme brute de synthèse préparée conformément au procédé décrit ci-dessus dans la présente description. Ladite zéolithe sous sa forme brute de synthèse comprend encore le structurant organique, lequel sera éliminé de la zéolithe par calcination conformément à ladite étape e), et au moins un type de cations alcalins et/ou alcalino-terreux, préférentiellement le sodium. Selon une deuxième variante, on utilise, pour ladite étape de mise en forme, une zéolithe IZM-2 sous sa forme calcinée, préparée conformément au procédé décrit ci-dessus dans la présente description.

La mise en forme de ladite zéolithe IZM-2, sous sa forme brute de synthèse ou calcinée, peut être réalisée par toute technique connue de l'Homme du métier. Elle peut en particulier être mélangée avec au moins une matrice minérale poreuse, généralement amorphe, par exemple avec une poudre humide de gel d'alumine. Le mélange est ensuite mis en forme par exemple par extrusion au travers d'une filière. La mise en forme peut être réalisée avec d'autres matrice que l'alumine, telles que par exemple la magnésie, les silices-alumines amorphes, les argiles naturelles (kaolin, bentonite, sepiolite, attapulgite), les silices, l'oxyde de titane, l'oxyde de bore, la zircone, les phosphates d'aluminium, les phosphates de titane, les phosphates de zirconium, le charbon et leurs mélanges. On préfère utiliser des matrices contenant de l'alumine sous toutes ses formes connues de l'Homme du métier et de manière encore plus préférée l'alumine gamma. On peut aussi avantageusement utiliser des mélanges d'alumine et de silice, des mélanges d'alumine et de silice-alumine. D'autres techniques que l'extrusion telles que le pastillage et la dragéification peuvent être utilisées. Les conditions de mise en forme de la zéolithe IZM-2, le choix de la matrice, éventuellement le broyage préalable de la zéolithe, le procédé de peptisation, l'ajout d'agents porogènes, le temps de malaxage, la pression d'extrusion si le catalyseur est mis sous forme d'extrudés, la vitesse et le temps de séchage, sont déterminés, pour chaque matrice, selon les règles bien connues de l'homme de métier, de manière à obtenir un catalyseur de préférence sous forme d'extrudés ou de billes.

Conformément à l'étape e) dudit deuxième mode de réalisation du procédé de préparation du catalyseur selon l'invention, le solide issu de ladite étape d) est soumis à une étape de calcination réalisée à une température comprise entre 250 et 600°C et de préférence entre 400 et 550°C. Ladite étape de calcination élimine le structurant organique occlus dans la porosité de la zéolithe IZM-2 brute de synthèse lorsque l'étape d) de mise en forme est réalisée sur la zéolithe IZM-2 sous sa forme brute de synthèse.

Conformément à l'étape f) dudit deuxième mode de réalisation du procédé de préparation du catalyseur selon l'invention, et selon une première variante, ladite zéolithe calcinée et mise en forme issue de ladite étape e) est soumise à au moins un échange ionique de manière à obtenir ladite zéolithe sous une forme hydrogène. Ladite zéolithe sous forme hydrogène contenue dans le catalyseur selon l'invention présente alors la formule $XO_2$ : $aY_2O_3$ où X, Y et a ont été définis plus haut dans la présente description. L'obtention de la zéolithe IZM-2 sous forme hydrogène est réalisée selon un protocole identique et dans des conditions opératoires semblables à celles décrites pour la mise en oeuvre de ladite étape a) dudit premier mode de réalisation du procédé de préparation du catalyseur selon l'invention.

Selon une deuxième variante de mise en oeuvre de ladite étape f) dudit deuxième mode de réalisation du procédé de préparation du catalyseur selon l'invention, ladite zéolithe calcinée et mise en forme issue de ladite étape e) est soumise à au moins un échange ionique de manière à obtenir ladite zéolithe sous une forme basique de formule

$XO_2 : aY_2O_3 : bM_nO$ où X, Y, M, n et a ont été définis plus haut dans la présente description et b est compris entre 0,005 et 0,5. Ladite zéolithe issue de ladite e) est soumise à un traitement par au moins un échange ionique avec des cations appartenant à la famille des métaux alcalins et alcalino-terreux, préférentiellement avec des cations choisis parmi les cations $Li^+$, $Na^+$, $K^+$, $Rb^+$, $Cs^+$, $Mg^{2+}$, $Ca^{2+}$ et $Ba^{2+}$, et très préférentiellement par des cations $Cs^+$. De manière avantageuse et conformément à ladite deuxième variante, il s'agit d'échanger les cations $Na^+$ présents dans la zéolithe IZM-2 issue de ladite étape e) par des cations $Cs^+$. Cet échange ionique est réalisé par toute technique connue de l'Homme du métier. Ladite deuxième variante de mise en oeuvre de ladite étape f) est réalisée en mettant en contact ladite zéolithe IZM-2, calcinée et mise en forme, issue de ladite étape e) avec une solution contenant au moins un précurseur du(es) métal(ux) appartenant à la famille des métaux alcalins et alcalino-terreux, préférentiellement au moins un précurseur du(es) métal(ux) choisi(s) parmi Li, Na, K, Rb, Cs, Mg, Ca et Ba, et très préférentiellement au moins un précurseur du césium. Parmi les précurseurs employés pour la mise en oeuvre de ladite deuxième variante, on peut citer les oxydes métalliques et leurs mélanges en toute proportion ainsi que des sels des métaux alcalins et alcalino-terreux, notamment des sels d'halogénures, de sulfates, de nitrates, de phosphates, de carbonates, d'oxalates, d'hydroxydes, d'acétates, d'alcoolates, de perchlorates, de carboxylates ou d'acétylacétonates. Lorsque la zéolithe issue de ladite étape e) est échangée avec des cations $Cs^+$, on utilise avantageusement de l'acétate de césium ou du nitrate de césium. Ces précurseurs peuvent être sous forme de poudre ou mis en forme, soluble ou insoluble dans le milieu réactionnel. Ledit traitement réalisé conformément à ladite deuxième variante de mise en oeuvre de ladite étape f) peut comprendre un ou plusieurs échanges ioniques successifs.

Conformément à ladite étape g) dudit deuxième mode de réalisation du procédé de préparation du catalyseur selon l'invention, la zéolithe échangée et mise en forme issue de ladite étape f) est soumise à un traitement par calcination réalisé à une température comprise entre 250 et 600°C.

Conformément audit deuxième mode de réalisation du procédé de préparation du catalyseur selon l'invention, ladite étape g) est avantageusement suivie d'une étape h) consistant à procéder à un traitement comprenant au moins un échange ionique des protons présents dans la zéolithe IZM-2 sous sa forme hydrogène de manière à obtenir un catalyseur comprenant une zéolithe IZM-2 sous forme basique. Ladite étape h) est avantageusement mise en oeuvre lorsqu'un catalyseur comprenant une zéolithe IZM-2 sous forme basique est recherchée pour une application particulière et que l'étape f) a conduit à l'obtention d'une zéolithe sous forme hydrogène. Ladite étape h) est réalisée selon un protocole et des conditions opératoires identiques à celles décrites pour la mise en oeuvre de ladite étape a') dudit premier mode de réalisation du procédé de préparation du catalyseur selon l'invention. Ladite étape h) est ensuite suivie d'une étape de calcination à une température comprise entre 250 et 600°C.

Conformément audit deuxième mode de réalisation dudit procédé de préparation du catalyseur selon l'invention, on procède avantageusement à un ou plusieurs traitement(s) connu(s) de l'Homme du métier visant à stabiliser, désaluminer ou passiver la zéolithe.

Lorsque le catalyseur selon l'invention comprend au moins un métal additionnel choisi parmi les métaux des groupes IIIB, IVA et IVB, le dépôt d'au moins dudit métal peut être effectué à tout moment de la préparation de l'un ou l'autre mode de réalisation du procédé de préparation décrit ci-dessus dans la présente description, soit avant la mise en forme, soit lors du mélange de la zéolithe et de la matrice, la zéolithe étant mélangée à l'ensemble constitué par le (s) précurseur(s) du(des)dit(s) métal(ux) et la matrice, soit, de manière préférée, après la mise en forme. Le(les)dit(s) métal(ux) ajouté(s) est(sont) généralement déposé(s), soit pratiquement totalement sur la zéolithe, soit en partie sur la zéolithe et en partie sur la matrice, soit, de préférence, pratiquement totalement sur la matrice, ceci s'effectuant, de la manière qui est connue de l'homme du métier, par le choix approprié des paramètres utilisés lors dudit dépôt, comme par exemple la nature du précurseur du(des)dit(s) métal(ux). Le dépôt d'au moins un métal choisi parmi les métaux des groupes IIIB, IVA et IVB peut être effectué par toutes les techniques de dépôt connues de l'Homme du métier, en particulier par la technique d'imprégnation à sec, d'imprégnation par excès ou d'échange ionique, de préférence par échange(s) ionique(s). Tous les précurseurs métalliques conviennent pour l'introduction dudit métal.

Le catalyseur selon l'invention est avantageusement utilisé pour la mise en oeuvre de différents procédés de transformation de charges hydrocarbonées.

En particulier, un autre objet de l'invention est un procédé de production d'au moins un ester alcoolique à partir d'au moins un composé appartenant à la famille des triglycérides et d'au moins un composé portant une fonction alcool réalisé en présence d'au moins un catalyseur selon l'invention tel qu'il est décrit ci-dessus dans la présente description. La réaction mise en jeu dans ledit procédé de production d'au moins un ester alcoolique est une réaction de transestérification des esters. Ledit composé portant une fonction alcool contient préférentiellement une seule fonction alcool. La mise en oeuvre dudit procédé conduit à la production d'une première phase organique essentiellement constituée d'au moins dudit ester alcoolique et d'une seconde phase organique essentiellement constituée de glycérol, ladite seconde phase étant appelée glycérine en raison des impuretés y présentes. L'ester alcoolique présent dans ladite première phase organique est utilisé en tant que carburant et plus précisément en tant que biodiesel. Il répond aux spécifications de la norme EN14214 (teneur en esters d'huiles végétales supérieure à 96,5%). Les spécifications actuelles imposent un rendement en ester alcoolique d'au moins 96,5 % poids, le rendement en ester alcoolique étant calculé comme étant

égal au pourcentage massique dudit ester dans ladite première phase organique laquelle contient éventuellement de façon très minoritaire le composé initial appartenant à la famille des triglycérides et / ou des composés intermédiaires, en particulier des composés (esters) appartenant à la famille des monoglycérides et des diglyécrides. De manière préférée, le composé initial appartenant à la famille des triglycérides ne représente pas plus de 0,2 % poids de ladite première phase organique, les composés appartenant à la famille des monoglycérides ne représente pas plus de 0,8 % poids de ladite première phase organique, les composés appartenant à la famille des diglycérides ne représente pas plus de 0,2 % poids de ladite première phase organique et la glycérine ne représentant pas plus de 0,25 % poids de ladite première phase organique. Ladite seconde phase organique contient de 95 à 99,9 % poids de glycérol et de préférence de 98 à 99,9 % poids de glycérol. Ladite seconde phase organique présente ainsi une pureté maximale en glycérol. Le procédé de production d'au moins un ester alcoolique selon l'invention conduit à la production d'un ester carburant répondant aux spécifications et une glycérine de pureté élevée en opérant en une ou deux étapes selon un procédé fonctionnant en continu ou en discontinu tel que décrit ci-après dans la présente description. Par ailleurs, l'ester alcoolique et le glycérol produits ne contiennent aucune impureté issue du catalyseur utilisé pour la mise en oeuvre du procédé de transestérification selon l'invention. Aussi, aucun traitement de purification de l'une ou l'autre phase organique n'est nécessaire.

La mise en oeuvre du catalyseur selon l'invention dans le procédé de production d'au moins un ester alcoolique est particulièrement avantageuse dès lors qu'il présente une bonne tenue au lessivage, laquelle est vérifiée par l'absence de traces métalliques provenant du catalyseur (silicium, aluminium, métal(ux) présent dans la zéolithe IZM-2 suite à une étape d'échange ionique) aussi bien dans l'ester alcoolique formé et présent dans ladite première phase organique que dans la glycérine produite et constituant ladite seconde phase organique. La recyclabilité ou durée de vie du catalyseur selon l'invention, évaluée expérimentalement dans le temps, est satisfaisante (plusieurs centaines d'heures) lorsqu'il est utilisé dans le procédé de production d'au moins un ester alcoolique selon l'invention.

Le procédé de production d'au moins un ester alcoolique selon l'invention à partir d'au moins un composé appartenant à la famille des triglycérides et d'au moins un composé portant une fonction alcool est mis en oeuvre selon différents modes de réalisation. Quel que soit le mode de réalisation choisi, ledit procédé est avantageusement opéré à une température comprise entre 130 et 220°C, à une pression inférieure à 10 MPa, plus précisément à la pression autogène, et avec un excès molaire du composé portant la fonction alcool par rapport à la stoechiométrie (composé appartenant à la famille des triglycérides / composé portant une fonction alcool). Selon le procédé de production d'au moins un ester alcoolique selon l'invention, le rapport molaire (composé portant une fonction alcool / composé appartenant à la famille des triglycérides) est supérieur à 3. De manière préférée, le procédé de production d'ester alcoolique selon l'invention est opéré en mettant en contact de 15 à 35 et de manière très préférée de 25 à 35 moles dudit composé portant une fonction alcool avec une mole dudit composé appartenant à la famille des triglycérides, soit un excès molaire compris entre 15 et 35, de préférence entre 25 et 35. En opérant à une température inférieure ou égale à 220°C, on obtient généralement un ester alcoolique de même couleur que le composé réactif appartenant à la famille des triglycérides et une glycérine incolore après décantation comme cela est décrit ci-après.

Un premier mode de réalisation du procédé de production d'au moins un ester alcoolique selon l'invention consiste à mettre en oeuvre ledit procédé en discontinu au moyen d'au moins un réacteur fermé (appelé encore réacteur "batch"). Conformément audit premier mode de réalisation, le procédé peut être mis en oeuvre en une ou deux étapes. Pour une mise en oeuvre en deux étapes, on réalise la première étape dans des conditions telles que le rendement en ester alcoolique à l'issue de ladite première étape est compris entre 85 % et 95 % poids, puis on refroidit l'effluent issu de ladite première étape en évaporant l'excès du composé portant ladite fonction alcool, on laisse décanter la phase formée de glycérine puis on procède à la deuxième étape en chauffant de nouveau le mélange réactionnel formé de la phase organique contenant l'ester alcoolique, le composé initial appartenant à la famille des triglycérides et les composés intermédiaires appartenant aux familles des monoglycérides et diglycérides, le composé portant ladite fonction alcool étant introduit de nouveau dans ledit mélange réactionnel. Ladite deuxième étape permet d'augmenter le rendement en ester alcoolique au-delà de 96,5 % poids. De manière préférée, le rendement en ester alcoolique est d'au moins 98% poids. La première et la deuxième étapes qui réalisent chacune une réaction de transestérification sont mises en oeuvre à une température comprise entre 130 et 220°C, la deuxième étape étant réalisée à une température avantageusement égale ou supérieure à celle de la première étape et à une pression correspondant à la pression autogène du milieu.

Pour une mise en oeuvre en une étape, les conditions opératoires sont adaptées de manière à ce que le rendement en ester alcoolique à l'issue de ladite étape soit d'au moins 96,5 % et de préférence d'au moins 98%. Pour obtenir un rendement aussi élevé en une seule étape, on opère généralement à une température et un rapport molaire (composé portant une fonction alcool / composé appartenant à la famille des triglycérides) plus élevés que les températures et les rapports molaires appliqués pour la mise en oeuvre du procédé en deux étapes. De manière avantageuse, pour un procédé en une étape, la température est comprise entre 175 et 220°C et le rapport molaire (composé portant une fonction alcool /composé appartenant à la famille des triglycérides) est supérieur à 30.

Un deuxième mode de réalisation du procédé de production d'au moins un ester alcoolique selon l'invention

consiste à mettre en oeuvre ledit procédé en continu c'est-à-dire que la production dudit ester alcoolique est continue. Ce mode de réalisation suppose la mise en oeuvre de plusieurs réacteurs autoclaves où se réalisent la réaction de transestérification et de plusieurs décanteurs réalisant la décantation des deux phases organiques, l'une comprenant l'ester alcoolique et l'autre formée de glycérine. Il est avantageux d'opérer la mise en oeuvre du procédé en continu en deux étapes : dans une première étape, on réalise dans un réacteur autoclave une réaction de transestérification dans des conditions telles que le rendement en ester alcoolique avoisine 85%, de préférence 90% poids puis on décante en évaporant le composé portant ladite fonction alcool et on refroidit et dans un deuxième réacteur autoclave on réalise une deuxième réaction de transestérification en ajoutant dans le milieu réactionnel une partie du composé portant la fonction alcool évaporé précédemment à la phase organique comprenant l'ester alcoolique issue de ladite première étape de manière à parfaire les performances du procédé de production d'au moins un ester alcoolique en augmentant le rendement dudit ester dans ladite deuxième étape. On évapore finalement l'excès du composé portant ladite fonction alcool et l'on sépare la glycérine et les esters (les mono - et diglycérides étant également des esters) par décantation. De manière préférée, on utilise pour chacune des deux étapes au moins un réacteur en lit fixe opérant à une température comprise entre 130 et 220°C, de préférence entre 150 et 180°C, à une pression comprise entre 1 et 7 MPa, une VVH (volume de la phase comportant au moins le composé de la famille des triglycérides / volume de catalyseur /h) comprise entre 0,1 et 3 $h^{-1}$, de préférence entre 0,3 et 2 $h^{-1}$ et un rapport massique (composé portant une fonction alcool / composé appartenant à la famille des triglycérides) variant de 3/1 à 0,1/1.

Une mise en oeuvre particulière et avantageuse du procédé en continu consiste à introduire le composé portant la fonction alcool de manière fractionnée au sein de chacun des réacteurs. On choisit généralement pour cette mise en oeuvre des réacteurs tubulaires. En particulier, il est avantageux d'introduire ledit composé portant la fonction alcool à deux niveaux d'un réacteur tubulaire : on introduit par exemple à l'entrée du réacteur la totalité du composé appartenant à la famille des triglycérides et les 2/3 volume de la quantité totale du composé portant la fonction alcool puis la quantité restante du composé portant la fonction alcool est introduite au niveau du dernier tiers de la hauteur du lit catalytique.

Conformément audit procédé de production d'au moins un ester alcoolique selon l'invention, le catalyseur utilisé comprend au moins une zéolithe IZM-2 soit sous forme hydrogène soit sous forme basique. Lorsque ladite zéolithe se trouve sous forme basique, ladite zéolithe a été échangée avec des cations appartenant à la famille des métaux alcalins et alcalino-terreux, préférentiellement avec des cations choisis parmi les cations $Li^+$, $Na^+$, $K^+$, $Rb^+$, $Cs^+$, $Mg^{2+}$, $Ca^{2+}$ et $Ba^{2+}$, et très préférentiellement par des cations $Cs^+$.

<u>Corps gras</u>

Conformément au procédé de production d'au moins un ester alcoolique selon l'invention, le composé appartenant à la famille des triglycérides est un corps gras. Les corps gras utilisés dans le procédé de l'invention correspondent à des substances naturelles ou élaborées, d'origine animale ou végétale, contenant majoritairement des triglycérides, couramment regroupés sous les termes d'huile et de graisses. Parmi les huiles utilisables, on peut citer toutes les huiles courantes, comme les huiles de palme (concrètes ou oléines), de soja, de palmiste, de coprah, de babassu, de colza (ancien ou nouveau), de tournesol (classique ou oléique), de maïs, de coton, les huiles d'arachide, de pourghère (*Jatropha curcas*), de ricin, de lin et de crambe et toutes les huiles issues par exemple du tournesol ou du colza par modification génétique ou hybridation ou encore provenant d'algues ou de micro-algues. On peut également utiliser des huiles de friture, d'équarrissage, des huiles animales variées, comme les huiles de poissons, de phoques, d'équarrissage, le suif, le saindoux, ou encore les graisses issues du traitement des eaux usées et même des graisses de volailles, car les esters fabriqués à partir de certains alcools comme l'alcool éthylique, isopropylique ou butylique, permettent de gagner plus de 10°C en point d'écoulement et par conséquent d'utiliser au départ des huiles plus saturées. Parmi les huiles utilisées, on peut encore indiquer des huiles partiellement modifiées par exemple par polymérisation ou oligomérisation, comme par exemple, les "standolies" d'huile de lin, de tournesol et les huiles végétales soufflées, les standolies étant connues de l'Homme du métier comme des huiles en partie polymérisées. Les huiles utilisées sont neutres ou acides, vierges ou recyclées. Selon le procédé de transestérification selon l'invention, la présence d'acide gras dans les huiles n'est pas a *priori* préjudiciable car les systèmes catalytiques à base de zéolithe IZM-2 échangée ou non sont également actifs pour l'estérification et transforment également les acides gras en esters. La valeur limite en acides gras libres contenus dans les huiles se situe à un indice d'acide voisin de 10 (l'indice d'acide étant défini comme la masse en mg de KOH nécessaire au dosage de tous les acides gras libres dans 1 g d'huile). L'opérabilité du procédé dans ces conditions est proche de celle définie avec une huile à faible indice d'acide (soit inférieure à 0,2 mg de KOH/g) généralement utilisée. Dans le cas d'huiles à très fort indice d'acide (proche de 10 mg de KOH/g); une des possibilités est de faire précéder la réaction de transestérification d'une réaction d'estérification des acides gras libres présents, soit en utilisant le même alcool que celui utilisé dans le procédé de transestérification en présence du catalyseur selon l'invention dans lequel la zéolithe IZM-2 est préférentiellement sous sa forme protonée, soit en utilisant de préférence de la glycérine, pour former un ester de glycérol total ou partiel, en utilisant le même catalyseur à base d'IZM-2 que celui employé pour la réaction de transestérification, à pression atmosphérique et de préférence sous vide et à des températures comprises

entre 150 et 220°C.

Lorsqu'on utilise des huiles de friture, qui constituent une matière première très bon marché pour produire un biodiesel, il est nécessaire d'éliminer du mélange réactionnel les polymères d'acides gras afin que le mélange d'esters réponde aux spécifications de la norme EN 14214.

<u>Alcool</u>

Conformément au procédé de production d'au moins un ester alcoolique selon l'invention, la nature du composé portant une fonction alcool joue un rôle dans l'activité de la réaction de transestérification et en conséquence sur la conversion des triglycérides. Ledit composé portant une fonction alcool est très préférentiellement un monoalcool et encore plus préférentiellement un monoalcool aliphatique renfermant de 1 à 18 atomes de carbone, de préférence de 1 à 12 atomes de carbone et de manière plus préférée de 1 à 5 atomes de carbone.

De préférence, le composé portant la fonction alcool est l'alcool méthylique (méthanol) qui est l'alcool qui conduit à la meilleure conversion des triglycérides. On utilise également avantageusement l'alcool éthylique et les alcools isopropylique, propylique, butylique, isobutylique et même amylique. Des alcools plus lourds tels que l'alcool éthyl-hexylique ou l'alcool laurique peuvent également être envisagés pour la mise en oeuvre du procédé de transestérification selon l'invention. On peut avantageusement ajouter aux alcools lourds de l'alcool méthylique qui facilite la réaction. Par ailleurs, lorsqu'on prépare l'ester éthylique, on peut utiliser un mélange d'alcool éthylique et méthylique comprenant de 1 à 50% en poids, de préférence de 1 à 10% en poids, d'alcool méthylique de manière à augmenter la conversion.

Un autre objet de l'invention porte sur un procédé de transformation d'au moins un composé aliphatique ayant de 1 à 18 atomes de carbone et portant une fonction alcool, ledit procédé étant réalisé en présence d'au moins un catalyseur selon l'invention, lequel comprend au moins une zéolithe IZM-2 préférentiellement sous sa forme hydrogène.

De manière préférée, ledit composé aliphatique portant une fonction alcool comprend de 1 à 12 atomes de carbone et de manière plus préférée de 1 à 6 atomes de carbone. De manière encore plus préférée, ledit composé aliphatique portant une fonction alcool est choisi parmi le méthanol et méthanol. Ledit composé aliphatique ayant de 1 à 18 atomes de carbone et portant une fonction alcool peut être linéaire ou ramifié. De préférence, il s'agit d'un monoalcool. L'utilisation d'alcools totalement anhydres n'est pas nécessaire pour la mise en oeuvre dudit procédé de transformation d'au moins un composé aliphatique ayant de 1 à 18 atomes de carbone et portant une fonction alcool.

Selon un premier mode de réalisation dudit procédé de transformation d'au moins un composé aliphatique ayant de 1 à 18 atomes de carbone et portant une fonction alcool, ladite transformation mise en jeu est une réaction de déshydratation au cours de laquelle ledit composé aliphatique portant une fonction alcool est déshydraté en oléfine(s) avec production d'eau. Conformément audit premier mode, on utilise préférentiellement l'éthanol comme composé aliphatique ayant une fonction alcool de manière à produire de l'éthylène. Les conditions opératoires pour la mise en oeuvre dudit procédé de transformation des alcools en oléfines sont les suivantes : la pression totale est inférieure à 2 MPa, de préférence comprise entre 0,05 et 1 MPa, la température est comprise entre 150 et 500 °C, de préférence comprise entre 200 et 350°C. La pph définie comme étant le débit massique d'introduction de la charge comprenant ledit composé aliphatique divisé par la masse de catalyseur varie en général entre 0,5 et 50 $h^{-1}$ et préférentiellement entre 1 et 25 $h^{-1}$. Un gaz inerte comme par exemple de l'azote ou un hydrocarbure léger peut être utilisé pour diluer la charge comprenant ledit composé aliphatique au niveau du catalyseur.

Ledit procédé de transformation des alcools en oléfines conformément audit premier mode de réalisation est avantageusement mis en oeuvre en lit fixe, mobile ou fluidisé. Hormis l'eau générée lors de la réaction de déshydratation, les éthers associés aux alcools introduits dans le réacteur peuvent être formés principalement dans le cas du méthanol et de l'éthanol. Lesdits éthers peuvent être avantageusement recyclés afin d'augmenter le rendement en oléfines.

Selon un deuxième mode de réalisation dudit procédé de transformation d'au moins un composé aliphatique ayant de 1 à 18 atomes de carbone et portant une fonction alcool, ladite transformation mise en jeu réalise simultanément dans un même réacteur la déshydratation dudit composé aliphatique en oléfine(s) et l'oligomérisation de(s)dite(s) oléfine (s). Il s'agit de produire des hydrocarbures qui seront incorporés dans le pool essence et / ou dans le pool diesel. Les conditions opératoires pour la mise en oeuvre d'une telle transformation sont telles que la température est comprise entre 250°C et 450°C, la pression totale est comprise entre 2 et 10 MPa et la PPH correspondant au débit massique d'introduction de la charge comprenant ledit composé aliphatique divisé par la masse de catalyseur est comprise entre 0,1 et 5 $h^{-1}$. L'augmentation de la pression pour la mise en oeuvre dudit deuxième mode par rapport audit premier mode de réalisation (déshydratation) favorise la formation de composés issus de l'oligomérisation des oléfines formées *in situ* dans le(s) réacteur(s). Le catalyseur à base d'IZM-2 selon l'invention est préférentiellement activé, de préférence en le soumettant à une calcination, préalablement à sa mise en contact dans le réacteur avec la charge comprenant ledit composé aliphatique aux conditions de réaction précitées. Un gaz inerte tel que l'azote ou un hydrocarbure léger est avantageusement utilisé pour diluer la charge au niveau du catalyseur.

Une variante dudit deuxième mode de réalisation du procédé de transformation selon l'invention consiste à séparer la mise en oeuvre de l'étape de déshydratation de celle de l'oligomérisation des oléfines formées dans l'étape

de déshydratation. Conformément à cette variante, un séparateur est avantageusement installé entre le réacteur utilisé pour la déshydratation des alcools en oléfines et le réacteur utilisé pour la transformation des oléfines en composés plus lourds. On peut réaliser la réaction de déshydratation et la réaction d'oligomérisation en présence d'un catalyseur à base d'une zéolithe IZM-2 selon l'invention ou réaliser la réaction de déshydratation en présence d'un catalyseur comprenant une zéolithe différente de la zéolithe IZM-2, une silice-alumine ou une alumine activée et réaliser la réaction d'oligomérisation en présence d'un catalyseur à base d'une zéolithe IZM-2 selon l'invention.

Quel que soit le mode de réalisation mis en oeuvre pour la transformation d'une charge comportant au moins un composé aliphatique portant une fonction alcool, la réaction de transformation de ladite charge par déshydratation ou par déshydratation puis oligomérisation peut-être réalisée dans tout type de réacteur connu de l'Homme du métier. Selon une première mise en oeuvre, ledit procédé de transformation de ladite charge est réalisé dans au moins un réacteur en lit fixe. Le catalyseur se situe alors préférentiellement dans un réacteur à lit radial afin de minimiser la perte de charge au travers du lit catalytique. Selon une deuxième mise en oeuvre, ledit procédé de transformation de ladite charge est réalisé dans au moins un réacteur en lit mobile. On peut utiliser un ou plusieurs réacteurs avec un ou plusieurs lits mobiles, avec injection étagée possible de la charge, couplés ou non à un système de régénération en continu.

Conformément audit procédé de transformation d'une charge comportant au moins un composé aliphatique portant une fonction alcool selon l'invention, l'effluent de réaction est conservé à sa pression de réaction, aux pertes de charge dans les équipements traversés près. L'effluent est refroidi en dessous du point de rosée de l'eau. S'agissant dudit deuxième mode de réalisation (déshydratation + oligomérisation), ledit effluent de réaction refroidi est introduit dans un dispositif permettant la séparation triphasique d'une phase gazeuse constituée notamment d'oléfines légères, d'un liquide organique (essence et gazole) et d'un liquide aqueux (eau, alcool non transformé, hydrocarbures solubilisés).

La présente invention a également pour objet un procédé d'oligomérisation d'une charge oléfinique consistant en la mise en contact de ladite charge avec au moins un catalyseur selon l'invention lequel comprend au moins une zéolithe IZM-2, préférentiellement sous sa forme hydrogène (forme protonée $H^+$). Ledit procédé permet la production de carburant, par exemple la production d'essence et/ou de kérosène et/ou de diesel et/ou plus généralement d'une coupe avec un point d'ébullition commençant à une température supérieure à 150°C. Ladite charge oléfinique contient des molécules hydrocarbonées ayant de 2 à 12 atomes de carbone par molécule et de manière préférée ayant de 2 à 7 atomes de carbone par molécule. Elle contient de 20 à 100 % en poids, et de préférence de 25 à 80% en poids d'oléfines.

Les oléfines présentes dans la charge oléfinique peuvent provenir de toute source contenant des oléfines ayant de 2 à 12 atomes de carbone par molécule, préférentiellement de 2 à 7 atomes de carbone par molécule. Lesdites oléfines peuvent provenir par exemple d'une unité de craquage catalytique en lit fluidisé (fluid catalytic cracking, FCC) et/ou d'une unité de vapocraquage, et/ou d'une unité de déshydrogénation de paraffines et/ou d'une unité de déshydratation polymérisante de méthanol en eau et oléfines légères.

La réaction d'oligomérisation peut être réalisée en phase liquide, en phase supercritique ou en phase gazeuse en présence du catalyseur selon l'invention dans lequel la zéolithe IZM-2 y présente se trouve préférentiellement sous sa forme hydrogène.

Ledit procédé d'oligomérisation selon l'invention est préférentiellement mis en oeuvre dans les conditions opératoires suivantes : la pression totale est comprise entre 0,1 et 10 MPa et préférentiellement entre 0,3 et 7 MPa, la température est comprise entre 40 et 600°C et préférentiellement entre 40°C et 400°C, la vitesse spatiale horaire exprimée en volume de charge introduite par volume de catalyseur et par heure est comprise entre 0,01 et 100 $h^{-1}$ et préférentiellement entre 0,1 et 20 $h^{-1}$.

La présente invention a également pour objet un procédé de production de phénylalcanes comprenant une réaction d'alkylation d'au moins un composé aromatique par au moins une oléfine linéaire, ledit procédé étant réalisé en présence d'au moins un catalyseur selon l'invention comprenant au moins une zéolithe IZM-2, préférentiellement sous sa forme acide (forme protonée $H^+$). De manière avantageuse, ledit catalyseur est disposé en lit fixe dans au moins une zone réactionnelle réalisant ladite réaction d'alkylation.

Selon un premier mode de mise en oeuvre dudit procédé de production de phénylalcanes, le composé aromatique est préférentiellement le benzène et l'oléfine linéaire utilisée contient 2 ou 3 atomes de carbone de manière à conduire à la production d'éthylbenzène ou de cumène. Ledit procédé de production d'éthylbenzène ou de cumène est réalisé par la mise en contact du benzène avec une charge renfermant soit de l'éthylène soit du propylène. La réaction d'alkylation est habituellement effectuée en phase liquide, en phase supercritique ou en phase gazeuse. Les conditions opératoires appliquées pour la mise en oeuvre dudit procédé de production d'éthylbenzène ou de cumène sont les suivantes : on opère à une température comprise entre 30 et 300°C, de préférence entre 150 et 300°C, sous une pression absolue comprise entre 0,1 et 10 MPa, de préférence comprise entre 2 et 7 MPa, avec un débit d'hydrocarbures liquides de 0,5 à 200 volumes par volume de catalyseur et par heure, et avec un rapport molaire benzène/oléfines compris entre 1 : 1 et 50 : 1, de préférence entre 3 : 1 et 7 : 1 pour le propylène et entre 7 : 1 et 12 : 1 pour l'éthylène.

Selon un deuxième mode de mise en oeuvre dudit procédé de production de phénylalcanes, le composé aromatique est préférentiellement le benzène et l'oléfine linaire utilisée comprend de 9 à 16 atomes de carbone par molécule, de préférence de 10 à 14 atomes de carbone de manière à produire des phénylalcanes linéaires ou alkylbenzènes linéaires (LAB). Ledit procédé de production de phénylalcanes est mis en oeuvre par la mise en contact du benzène avec une charge comprenant au moins une oléfine linéaire dans au moins une zone réactionnelle contenant ledit catalyseur selon l'invention fonctionnant préférentiellement en lit fixe. Les conditions opératoires appliquées pour la mise en oeuvre dudit procédé de production de phénylalcanes selon l'invention sont choisies par l'Homme du métier. La zone réactionnelle est opérée à une température avantageusement inférieure à 400°C, de préférence inférieure à 350 °C et de manière très préférée inférieure à 300 °C et sous une pression de 1 à 10 MPa, de préférence de 2 à 6 MPa, avec un débit d'hydrocarbures liquides (vitesse spatiale) de 0,5 à 80, de préférence de 0,5 à 50, volumes par volume de catalyseur et par heure. Le ratio molaire benzène / oléfines est avantageusement compris entre 1 et 50, de préférence entre 10 et 30.

La présente invention a également pour objet un procédé de dismutation du toluène pour produire du benzène et des xylènes réalisé en présence d'au moins un catalyseur selon l'invention comprenant au moins une zéolithe IZM-2, préférentiellement sous forme acide (forme protonée $H^+$). Ledit catalyseur se révèle très efficace pour ladite utilisation, car il se révèle être particulièrement actif, sélectif et stable.

Les conditions opératoires appliquées pour la mise en oeuvre dudit procédé de dismutation selon l'invention sont généralement les suivantes : une température comprise entre 250 et 650°C et de préférence entre 350 et 550°C ; une pression comprise entre 1 et 6 MPa et de préférence entre 2 et 4,5 MPa ; une vitesse spatiale d'alimentation, exprimée en kilogramme de charge introduite par kilogramme de catalyseur et par heure, comprise entre 0,1 et 10 $h^{-1}$ et de préférence entre 0,5 et 4 $h^{-1}$ ; un rapport molaire hydrogène sur hydrocarbures compris entre 2 et 20 et de préférence entre 3 et 12 mol/mol.

Les exemples qui suivent illustrent la présente invention sans en limiter la portée.

**Exemples**

Exemple 1 préparation du dibromure de 1,6-bis(méthylpiperidinium)hexane pour la préparation des zéolithes IZM-2 (Z1 et Z2).

50 g de 1,6-dibromohexane (0,20 mole, 99%, Alfa Aesar) sont ajoutés dans un ballon de 1 L contenant 50 g de N-méthylpipéridine (0,51 mole, 99%, alfa Aesar) et 200 ml d'éthanol. Le milieu réactionnel est agité et porté à reflux pendant 5 h. Le mélange est ensuite refroidi à température ambiante puis filtré. Le mélange est versé dans 300 ml de diéthyléther froid puis le précipité formé est filtré et lavé avec 100 ml de diéthyléther. Le solide obtenu est recristallisé dans un mélange éthanol/éther. Le solide obtenu est séché sous vide pendant 12 h. On obtient 71 grammes d'un solide blanc (soit un rendement de 80 %). Le produit obtenu possède le spectre 1H RMN attendu. 1H RMN (D2O, ppm/TMS) : 1,27 (4H,m) ; 1,48 (4H,m) ; 1,61 (4H,m) ; 1,70 (8H,m) ; 2,85 (6H,s) ; 3,16 (12H,m).

Exemple 2 : préparation d'une zéolithe IZM-2 (Z1)

30 g d'une suspension colloïdale de silice, connue sous le terme commercial Ludox AS-40 commercialisée par Aldrich, est incorporée dans une solution composée de 0,158 g d'aluminate de sodium (Carlo Erba), 2,433 g de soude (Prolabo), 14,7333 g de dibromure de 1,6-bis(méthylpiperidinium)hexane préparé selon l'exemple 1 et de 101,561 g d'eau déionisée. La composition molaire du mélange est la suivante : $SiO_2$ ; 0,005 $Al_2O_3$ ; 0,17 $Na_2O$ ; 0,17 1,6-bis (méthylpiperidinium)hexane; 33,33 $H_2O$. Le mélange est agité vigoureusement pendant une demi-heure. Le mélange est ensuite transféré, après homogénéisation, dans un autoclave. L'autoclave est chauffé pendant 6 jours à 170°C sous agitation (200 tours / min). Le produit cristallisé obtenu est filtré, lavé à l'eau déionisée (pour atteindre un pH neutre) puis séché une nuit à 100°C. Le solide est ensuite introduit dans un four à moufle où est réalisée la calcination : le cycle de calcination comprend une montée en température jusqu'à 200°C, un palier à 200°C maintenu durant 2 heures, une montée en température jusqu'à 550°C suivi d'un palier à 550°C maintenu durant 8 heures puis un retour à la température ambiante.

Le solide calciné a été analysé par diffraction des rayons X et identifié comme étant constitué de zéolithe IZM-2. Ce solide correspond à une zéolithe IZM-2 échangée avec $Na^+$. Il est noté Z1.

Exemple 3 : préparation d'un catalyseur C1 comprenant une zéolithe IZM-2 sous forme hydrogène et une matrice aluminique (invention)

Le catalyseur C1 comprenant une zéolithe IZM-2 et de l'alumine est fabriqué de la façon suivante : 7 g de la zéolithe Z1 sont mélangés à 43 g d'une matrice composée d'un gel d'alumine commercialisé sous le nom SB3 par la société Condéa Chemie Gmbh. Ce mélange de poudre a été ensuite mélangé à une solution aqueuse contenant de l'acide nitrique à 66% en poids (7% en poids d'acide par gramme de gel sec) puis malaxé pendant 15 minutes. La pâte malaxée est ensuite extrudée à travers une filière de diamètre 1,2 mm. Les extrudés sont ensuite calcinés à 500°C

(rampe de montée de 5°C/min) durant 2 heures en lit traversé sous air sec (1 l air / h / g de solide).

Les extrudés ainsi calcinés sont échangés trois fois au contact d'une solution de nitrate d'ammonium afin d'obtenir la zéolithe IZM-2 sous sa forme acide : 50 g d'extrudés sont mis au contact de 400 ml d'une solution 10N de nitrate d'ammonium et le mélange est porté à reflux sous agitation durant 4 heures. Le mélange est ensuite filtré puis les extrudés rincés avec 800 ml d'eau distillée et séchés en couche mince durant une nuit en étuve à 150°C. Cette opération est effectuée trois fois. Après le troisième échange, les extrudés sont ensuite calcinés à 450°C (rampe de montée de 5°C/min) durant deux heures en lit traversé sous air sec (2 l air / h / gramme de solide). On obtient ainsi le catalyseur C1 dont la teneur pondérale en sodium de la zéolithe IZM-2, mesurée par adsorption atomique sur les extrudés échangés et calcinés, est inférieure à 50 ppm. Le catalyseur C1 comprend une zéolithe IZM-2 sous forme acide ($H^+$) ayant la composition molaire $SiO_2$ : 0,0057 $Al_2O_3$ soit un rapport Si/Al égal à 88. Ledit catalyseur C1 est constitué de 19 % poids de zéolithe IZM-2 sous forme acide et de 81 % poids d'alumine.

<u>Exemple 4: préparation d'un catalyseur C2 comprenant une zéolithe IZM-2 échangée au césium et une matrice aluminique (invention).</u>

Le catalyseur C2 comprenant une zéolithe IZM-2 et de l'alumine est fabriqué de la façon suivante : 7 g de la zéolithe Z1 sont mélangés à 43 g d'une matrice composée de gel d'alumine commercialisé sous le nom SB3 par la société Condéa Chemie Gmbh. Ce mélange de poudre a été ensuite mélangé à une solution aqueuse contenant de l'acide nitrique à 66% en poids (7% en poids d'acide par gramme de gel sec) puis malaxé pendant 15 minutes. La pâte malaxée est ensuite extrudée à travers une filière de diamètre 1,2 mm. Les extrudés sont ensuite calcinés à 500°C (rampe de montée de 5°C/min) durant 2 heures en lit traversé sous air sec (1 l air / h / g de solide).

La forme basique de la zéolithe IZM-2 échangée avec du césium présente dans les extrudés est préparée comme suit : 50 g d'extrudés sont mis au contact de 400 ml d'une solution 0,3 M de nitrate de césium et le mélange est porté à reflux sous agitation durant 4 heures. Cette opération est répétée jusqu'à ce que le milieu réactionnel ait le pH de la solution initiale de nitrate de césium. Le mélange est ensuite filtré puis les extrudés rincés avec 800 ml d'eau distillée et séchés en couche mince durant une nuit en étuve à 150°C. Cette opération est effectuée trois fois. Après le troisième échange, les extrudés sont ensuite calcinés à 450°C (rampe de montée de 5°C/min) durant deux heures en lit traversé sous air sec (2 l air / h / g de solide). On obtient ainsi le catalyseur C2 dont la teneur pondérale en sodium de la zéolithe IZM-2, mesurée par adsorption atomique sur les extrudés échangés et calcinés, est inférieure à 50 ppm. Le catalyseur C2 comprend une zéolithe IZM-2 sous forme basique échangée au césium ayant la composition $SiO_2$ : 0,0057 $Al_2O_3$ : 0,17 $Cs_2O$ soit un rapport Si/Al égal à 88. Le catalyseur C2 est constitué de 19 % poids de zéolithe IZM-2 sous forme basique (Cs) et de 81 % poids d'alumine.

<u>Exemple 5 : préparation d'une zéolithe IZM-2 (Z2)</u>

20,115 g d'une suspension colloïdale de silice, connue sous le terme commercial Ludox AS-40 commercialisée par Aldrich, est incorporée dans une solution composée de 0,422 g d'aluminate de sodium (carlo erba), 1,48 g de soude (prolabo), 9,879 g de dibromure de 1,6-bis(méthylpiperidinium)hexane préparé selon l'exemple 1 et de 68,104 g d'eau déionisée. La composition molaire du mélange est la suivante : $SiO_2$ ; 0,017 $Al_2O_3$ ; 0,17 $Na_2O$ ; 0,17 1,6-bis(méthylpiperidinium)hexane ; 33,33 $H_2O$. Le mélange est agité vigoureusement pendant une demi-heure. Le mélange est ensuite transféré, après homogénéisation, dans un autoclave. L'autoclave est chauffé pendant 8 jours à 170°C sous agitation (200 tours / min). Le produit cristallisé obtenu est filtré, lavé à l'eau déionisée (pour atteindre un pH neutre) puis séché une nuit à 100°C. Le solide est ensuite introduit dans un four à moufle où est réalisée la calcination : le cycle de calcination comprend une montée en température jusqu'à 200°C, un palier à 200°C maintenu durant 2 heures, une montée en température jusqu'à 550°C suivi d'un palier à 550°C maintenu durant 8 heures puis un retour à la température ambiante.

Le solide calciné a été analysé par diffraction des rayons X et identifié comme étant constitué de zéolithe IZM-2. Ce solide correspond à une zéolithe IZM-2 échangée avec Na+. Il est noté Z2.

<u>Exemple 6: préparation d'un catalyseur C3 comprenant une zéolithe IZM-2 et une matrice aluminique (invention)</u>

Le catalyseur C3 comprenant une zéolithe IZM-2 et de l'alumine est fabriqué de la façon suivante : 7 g de la zéolithe IZM-2 (Z2) sont mélangés à 43 g d'une matrice composée de gel d'alumine commercialisé sous le nom SB3 par la société Condéa Chemie Gmbh. Ce mélange de poudre a été ensuite mélangé à une solution aqueuse contenant de l'acide nitrique à 66% en poids (7% en poids d'acide par gramme de gel sec) puis malaxé pendant 15 minutes. La pâte malaxée est ensuite extrudée à travers une filière de diamètre 1,2 mm. Les extrudés sont ensuite calcinés à 500°C (rampe de montée de 5°C/min) durant 2 heures en lit traversé sous air sec (1 l air / h / gramme de solide).

Les extrudés ainsi calcinés sont échangés trois fois au contact d'une solution de nitrate d'ammonium afin d'obtenir la zéolithe IZM-2 sous sa forme acide : 50 g d'extrudés sont mis au contact de 400 ml d'une solution 10N de

nitrate d'ammonium et le mélange est porté à reflux sous agitation durant 4 heures. Le mélange est ensuite filtré puis les extrudés rincés avec 800 ml d'eau distillée et séchés en couche mince durant une nuit en étuve à 150°C. Cette opération est effectuée trois fois. Après le troisième échange, les extrudés sont ensuite calcinés à 450°C (rampe de montée de 5°C/min) durant deux heures en lit traversé sous air sec (2 l air / h / gramme de solide).

On obtient ainsi le catalyseur C3 dont la teneur pondérale en sodium de la zéolithe IZM-2, mesurée par adsorption atomique sur les extrudés échangés et calcinés, est inférieure à 50 ppm. Le catalyseur C3 comprend une zéolithe IZM-2 sous forme acide ($H^+$) ayant la composition molaire $SiO_2$: 0,0263 $Al_2O_3$ soit un rapport Si/Al égal à 19. Ledit catalyseur C3 est constitué de 19 % poids de zéolithe IZM-2 sous forme acide et de 81 % poids d'alumine.

<u>Exemple 7 : Transestérification d'huiles végétales (huile de colza) par le méthanol en présence du catalyseur C1 (invention).</u>

Cet exemple est réalisé en mettant en oeuvre le procédé de transestérification en discontinu au moyen d'un réacteur fermé. L'huile utilisée est de l'huile de colza dont l'indice d'acide est mesuré comme étant égal à 0,1 mg KOH / g huile et dont la composition en acides gras est donnée dans le tableau 2. Le chiffre qui suit les 2 points (:) dans le sigle Cx:y figurant dans la colonne "nature de la chaîne grasse" correspond au nombre d'insaturation (double liaison) des molécules présentes dans chacun des glycérides d'acides gras et le chiffre qui suit la lettre C correspond au nombre d'atomes de carbone des molécules présentes dans chacun des glycérides d'acides gras.

Tableau 2 : Composition de l'huile de colza.

| Glycéride d'acides gras | Nature de la chaîne grasse | % en masse |
|---|---|---|
| Palmitique | C16:0 | 5 |
| Palmitoléique | C16 :1 | < 0,5 |
| Stéarique | C18 :0 | 2 |
| Oléique | C18 :1 | 59 |
| Linoléique | C18:2 | 21,5 |
| Linolénique | C18:3 | 9 |
| Arachidique | C20:0 | < 0,5 |
| Gadoléique | C20 :1 | 1 |
| Béhénique | C22 :0 | < 0,5 |
| Erucique | C22:1 | < 1 |

On introduit dans un réacteur fermé à température ambiante 25 g d'huile de colza, 17 g de méthanol et 1 g de catalyseur C1. Le ratio massique méthanol/ huile est donc de 0,68, ce qui correspond à un ratio molaire de 19. Le réacteur est ensuite fermé, agité (300 trs/min) et chauffé à 210°C à l'aide d'un agitateur magnétique chauffant. La température du milieu réactionnel est stabilisée à 210°C après 42 minutes de chauffe. La pression est la pression autogène de l'alcool à la température de travail. Le suivi de la réaction est commencé lorsque la température du milieu réactionnel a atteint la consigne. Des prélèvements sont effectués de manière régulière afin de suivre l'avancement de la réaction dans le réacteur. Après 6 h de réaction, l'agitation est arrêtée et le réacteur laissé à refroidir jusqu'à température ambiante. Les prélèvements effectués sont lavés par une solution aqueuse saturée en NaCl, puis après décantation, la phase organique supérieure est analysée par chromatographie par perméation de gel (GPC). Le tableau suivant résume les résultats obtenus.

| | Prélèvements (en h) | 0[b] | 2 | 4 | 6 |
|---|---|---|---|---|---|
| % pds dans la phase organique[a] | Triglycérides | 40 | 20 | 5 | 1 |
| | Diglycérides[c] | 12 | 8 | 3 | 2 |
| | Monoglycéride | 5 | 8 | 5 | 3 |
| | Esters méthyliques d'huile végétale | 43 | 64 | 87 | 94 |

[a] déterminé par GPC
[b] t=0 lorsque le milieu réactionnel est à la température voulue
[c] % représentant les diglycérides et stérols

On note que la conversion des triglycérides commence alors que le milieu réactionnel n'a pas atteint 210°C (43% d'esters à t0).

A l'issue de la première étape de réaction, on procède à une décantation en évaporant le méthanol excédentaire et en refroidissant la phase organique comprenant les esters méthyliques d'huile végétale. Ladite phase organique (25 g), contenant 94% d'esters, 3% de monoglycérides, 2% de diglycérides et 1% de triglycérides est introduite dans un réacteur fermé, en présence de 17 g de méthanol et 1 g du catalyseur C1 afin d'obtenir un biodiesel aux spécifications. Le réacteur est ensuite fermé, agité (300 trs/min) et chauffé à 210°C à l'aide d'un agitateur magnétique chauffant. La température du milieu réactionnel est stabilisée à 210°C après 42 minutes de chauffe. Des prélèvements sont effectués de manière régulière afin de suivre l'avancement de la réaction dans le réacteur. Après 6 h de réaction, l'agitation est arrêtée et le réacteur laissé à refroidir jusqu'à température ambiante. Les prélèvements effectués ainsi que l'effluent final sont lavés par une solution aqueuse saturée en NaCl, puis après décantation, la phase organique supérieure est analysée par chromatographie par perméation de gel (GPC). Les résultats obtenus sont reportés dans le tableau suivant.

| | Prélèvements (en h) | 0[b] | 2 | 4 | 6 |
|---|---|---|---|---|---|
| % pds dans la phase organique[a] | Triglycérides | 1 | 0,4 | 0,2 | 0,1 |
| | Diglycérides[c] | 2 | 1 | 0,5 | 0,1 |
| | Monoglycéride | 3 | 1 | 0,8 | 0,5 |
| | Esters méthyliques d'huile végétale | 94 | 97,6 | 98,5 | 99,3 |

[a] déterminé par GPC
[b] t=0 lorsque le milieu réactionnel est à la température voulue
[c] % représentant les diglycérides et stérols

Le biocarburant ainsi obtenu répond aux normes en vigueur sur les teneurs en tri-, di- et monoglycérides, ainsi que sur la teneur en esters.

Exemple 8 : Transestérification d'huiles végétales (huile de colza) par le méthanol en présence du catalyseur C2 (invention).

Cet exemple est réalisé en mettant en oeuvre le procédé de transestérification en discontinu au moyen d'un réacteur fermé. L'huile utilisée est de l'huile de colza ayant les mêmes caractéristiques et la même composition que celle utilisée pour l'exemple 7.

On introduit dans un réacteur fermé à température ambiante 25 g d'huile de colza, 20 g de méthanol et 1 g de catalyseur C2. Le ratio massique méthanol/ huile est donc de 0,8, ce qui correspond à un ratio molaire de 22. Le réacteur est ensuite fermé, agité (500 trs/min) et chauffé à 160°C à l'aide d'un agitateur magnétique chauffant. La température du milieu réactionnel est stabilisée à 160°C après 20 minutes de chauffe. La pression est la pression autogène de l'alcool à la température de travail. Le suivi de la réaction est commencé lorsque la température du milieu réactionnel a atteint la consigne. Des prélèvements sont effectués de manière régulière afin de suivre l'avancement de la réaction. Après 5 h de réaction, l'agitation est arrêtée et le réacteur laissé à refroidir jusqu'à température ambiante. Les prélèvements effectués sont lavés par une solution aqueuse saturée en NaCl, puis après décantation, la phase organique supérieure est analysée par chromatographie par perméation de gel (GPC). Le tableau suivant résume les résultats obtenus.

| | Prélèvements (en h) | 0[b] | 1 | 3 | 5 |
|---|---|---|---|---|---|
| % pds dans la phase organique[a] | Triglycérides | 70 | 20 | 5 | 1 |
| | Diglycérides[c] | 5 | 12 | 6 | 2 |
| | Monoglycéride | 2 | 8 | 10 | 4 |
| | Esters méthyliques d'huile végétale | 23 | 60 | 79 | 93 |

[a] déterminé par GPC
[b] t=0 lorsque le milieu réactionnel est à la température voulue
[c] % représentant les diglycérides et stérols

La conversion des triglycérides commence alors que le milieu réactionnel n'a pas atteint 160°C (23% d'esters à t0).

A l'issue de la première étape de réaction, on procède à une décantation en évaporant le méthanol excédentaire et en refroidissant la phase organique comprenant les esters méthyliques d'huile végétale. Ladite phase organique (25 g), contenant 93% d'esters, 4% de monoglycérides, 2% de diglycérides et 1% de triglycérides est introduite dans un réacteur fermé, en présence de 20 g de méthanol et 1 g du catalyseur C2 afin d'obtenir un biodiesel aux spécifications. Le réacteur est ensuite fermé, agité (300 trs/min) et chauffé à 170°C à l'aide d'un agitateur magnétique chauffant. La température du milieu réactionnel est stabilisée à 170°C après 30 minutes de chauffe. Des prélèvements sont effectués de manière régulière afin de suivre l'avancement de la réaction dans le réacteur. Après 4 h de réaction, l'agitation est arrêtée et le réacteur laissé à refroidir jusqu'à température ambiante. Les prélèvements effectués ainsi que l'effluent final sont lavés par une solution aqueuse saturée en NaCl, puis après décantation, la phase organique supérieure est analysée par chromatographie par perméation de gel (GPC). Les résultats obtenus sont reportés dans le tableau suivant.

| | Prélèvements (en h) | 0[b] | 1 | 2 | 4 |
|---|---|---|---|---|---|
| % pds dans la phase organique[a] | Triglycérides | 1 | 0,5 | 0,2 | 0,1 |
| | Diglycérides[c] | 2 | 0,8 | 0,2 | 0,1 |
| | Monoglycéride | 4 | 2 | 1 | 0,6 |
| | Esters méthyliques d'huile végétale | 93 | 96,7 | 98,6 | 99,2 |

[a] déterminé par GPC
[b] t=0 lorsque le milieu réactionnel est à température
[c] % représentant les diglycérides et stérols

Le biocarburant ainsi obtenu répond aux normes en vigueur sur les teneurs en tri-, di- et monoglycérides, ainsi que sur la teneur en esters.

<u>Exemple 9 : déshydratation d'alcools à pression atmosphérique en présence du catalyseur C3 (invention)</u>

On procède dans cet exemple à deux tests catalytiques différents : le premier étant réalisé avec une charge constituée d'éthanol et le second avec une charge constituée de pentanol.

Pour chacun des tests, une unité pilote en lit fixe traversé est chargée avec 1,5 g de catalyseur C3. Avant de procéder à chacun des tests, le catalyseur C3 est activé en le soumettant à une calcination à 550°C sous air durant 2 h.

Pour la mise en oeuvre de chacun des tests, on dilue l'éthanol respectivement le pentanol avec de l'azote de sorte que le rapport molaire $N_2$/alcool soit égal à 4. La charge diluée à l'azote est injectée sur le catalyseur C3 placé dans le réacteur en lit fixe traversé. Les conditions opératoires pour chacun des tests sont données dans le tableau 3. Chaque test est réalisé à la pression atmosphérique (0,1 MPa). A la sortie du réacteur, on procède à la séparation de la phase gaz comprenant de l'azote, de la phase liquide organique (si elle existe) et de la phase liquide aqueuse. Dans le cas du premier test catalytique où la charge contient l'éthanol, l'oléfine produite est l'éthylène que l'on retrouve dans la phase gaz et dans le cas du second test catalytique où la charge contient le pentanol, les oléfines produites sont les pentènes que l'on retrouve dans la phase liquide organique. Dans le tableau 3, sont indiqués, en plus des conditions opératoires, le taux de conversion de l'alcool introduit ainsi que le rendement en oléfines.

Tableau 3 : Conditions opératoires et performances du catalyseur C3 dans la déshydratation d'alcools à pression atmosphérique

| alcool présent dans la charge | | éthanol | pentanol |
|---|---|---|---|
| | | | |
| Conditions opératoires | | | |
| | T(°C) | 250°C | 200°C |
| | pph ($h^{-1}$) | 2 | 10 |
| | TOS* (h) | 24 | 24 |
| | | | |
| Conversion (%) | | 99,7 | 100 |
| Rendement en oléfines (%) | | 58 | 78,5 |
| * TOS (time on stream) représente le temps de contact du catalyseur avec la charge. La conversion et le rendement sont calculés comme suit. | | | |

Conversion = (débit massique d'alcool$_{entrée}$ – débit massique d'alcool$_{sortie}$) / débit massique d'alcool$_{entrée}$.

Rendement = (débit massique d'oléfine $_{sortie}$) / débit massique d'alcool $_{entrée}$

Ces résultats démontrent que le catalyseur C3 selon l'invention est très actif pour la transformation d'alcools en oléfine(s) qui sont produites avec un rendement optimal.

Exemple 10 : transformation d'alcools sous pression en présence du catalyseur C3 (invention)

On procède dans cet exemple à deux tests catalytiques différents : le premier étant réalisé avec une charge constituée d'éthanol et le second avec une charge constituée de pentanol.

Pour chacun des tests, une unité pilote en lit fixe traversé est chargée avec 1,5 g de catalyseur C3. Avant de procéder à chacun des tests, le catalyseur C3 est activé en le soumettant à une calcination à 550°C sous air durant 2 h.

Pour la mise en oeuvre de chacun des tests, on dilue l'éthanol respectivement le pentanol avec de l'azote de sorte que le rapport molaire $N_2$/alcool soit égal à 4. La charge diluée à l'azote est injectée sur le catalyseur C3 placé dans le réacteur en lit fixe traversé. Les conditions opératoires pour chacun des tests sont données dans le tableau 4. A la sortie du réacteur, on procède à la séparation de la phase gaz, de la phase liquide organique et de la phase liquide aqueuse. Dans le tableau 4 sont indiqués, en plus des conditions expérimentales, le bilan massique des produits organiques récupérés. Ceux-ci sont divisés en trois catégories, les gaz, les liquides de faibles points d'ébullition (bp < 150°C) et les liquides de points d'ébullition élevés (bp > 150°C).

tableau 4 : Conditions opératoires et performances du catalyseur C3 dans la transformation d'alcools sous pression

| alcool présent dans la charge | | éthanol | pentanol |
|---|---|---|---|
| | | | |
| Conditions opératoires | | | |
| | T (°C) | 330 | 300 |
| | P (Mpa) | 3 | 3 |
| | pph ($h^{-1}$) | 1,2 | 1,5 |
| | TOS (h) | 5 | 5 |
| | | | |

(suite)

| Conditions opératoires | | | |
|---|---|---|---|
| Conversion (%) | | 100 | 99,8 |
| | | | |
| Répartition des produits dans l'effluent de sortie (% massique) | | | |
| | qaz | 28,1% | 13,2% |
| | liquide (bp*<150°C) | 34,6% | 29,8% |
| | liquide (bp*>150°C) | 37,3% | 57% |
| * bp (boiloing point) : point d'ébullition | | | |

La conversion est calculée de la même manière que dans l'exemple 9.

Les résultats figurant dans le tableau 4 démontrent que le catalyseur C3 selon l'invention est très actif dans la transformation d'alcools sous pression et conduit à des produits aisément incorporables dans le pool essence (phase liquide ayant un point d'ébullition inférieur à 150°C) et dans le pool diesel (phase liquide ayant un point d'ébullition supérieur à 150°C).

<u>Exemple 11 : évaluation catalytique du catalyseur C3 dans un procédé d'oligomérisation (invention)</u>

Une coupe C4 oléfinique issue d'une unité de craquage catalytique est séchée sur un tamis moléculaire de type 13X pour éliminer les traces de soufre et d'eau. La composition de la charge à l'issue de ces traitements est la suivante:

| Composition de la charge (% poids) | |
|---|---|
| Isobutane | 29,3 |
| n-butane | 8,8 |
| isobutène | 18,3 |
| 1-butène | 13,9 |
| ∑ 2-butènes | 29,7 |

Le catalyseur C3 selon l'invention est chargé dans un réacteur en lit fixe et testé pour la réaction d'oligomérisation de la charge décrite ci dessus.

Les conditions opératoires appliquées dans le réacteur d'oligomérisation sont telles que la pression est égale à 5,5 MPa et le VVH (débit volumique de charge / volume de catalyseur) est égale à 1 $h^{-1}$. Le catalyseur est testé à deux températures : d'abord à 200°C puis à 230°C.

Les conversions des oléfines par le catalyseur C3 aux températures d'oligomérisation étudiées de 200°C et 230°C, et plus particulièrement les conversions de l'isobutène et des n-butènes, à savoir les 1-butène et 2-butènes, sont reportées dans le tableau 5. La conversion est définie comme le rapport entre la quantité d'oléfines convertie et la quantité d'oléfines totale présente initialement dans la charge.

Le catalyseur C3 est un catalyseur très actif pour la réaction d'oligomérisation d'une charge oléfinique. En effet, à 200 et 230°C, la conversion de l'isobutène de la charge est totale (100%). La conversion des butènes, équivalente à la conversion des butènes normaux et de l'isobutène, est élevée et améliorée par augmentation de la température.

Tableau 5 : performance du catalyseur C3 en oligomérisation d'une coupe C4 oléfinique

| Température | 200°C | 230°C |
|---|---|---|
| Conversion butène (%pds.) | 78,6 | 89,2 |
| Conversion isobutène (%pds.) | 100 | 100 |

<u>Exemple 12 : production d'alkylbenzènes linéaires en présence du catalyseur C1 (invention)</u>

50 $cm^3$ du catalyseur C1 qui comprend une zéolithe IZM-2 sous forme hydrogène sont placés dans une zone réactionnelle alimentée par une charge constituée de 72% poids de benzène et 28% poids de dodécène-1. Les conditions opératoires pour l'alkylation du benzène par le dodécène-1 sont les suivantes :

- température : 135°C
- pression : 4 MPa
- WH = 1 $h^{-1}$ ($cm^3$ charge benzène+dodécène-1 par $cm^3$ de catalyseur et par heure)
- rapport molaire benzène/dodécène-1 : 30

Les performances catalytiques sont reportées dans le tableau 6 où figurent le nombre d'heures de fonctionnement à une conversion supérieure à 95% et la linéarité des alkylbenzènes formés.

Tableau 6 : Performances catalytiques du catalyseur C1 en alkylation du benzène

| | C1 |
|---|---|
| Nombre d'heures / conversion >95% | 35 |
| linéarité des alkyl-benzène (%) | 91,8 |

La conversion mesure le taux de transformation du dodécène-1.

Conversion=(quantité dodécène-$1_{entrée}$ - quantité dodécène-$1_{sortie}$) / quantité dodécène-$1_{entrée}$ Le catalyseur C1 selon l'invention est particulièrement actif lorsqu'il est utilisé pour la mise en oeuvre d'un procédé de production d'alkylbenzènes linéaires. Il favorise la production d'alkylbenzènes linaires.

<u>Exemple 13 : procédé de dismutation du toluène en présence du catalyseur C1 (invention).</u>

Le catalyseur C1 qui comprend une zéolithe IZM-2 sous forme hydrogène est introduit dans une zone réactionnelle où il est d'abord réduit sous hydrogène à 450°C pendant 2h. Puis une charge constituée de 100% poids de toluène est ensuite introduite dans ladite zone. Les conditions opératoires pour la dismutation du toluène sont les suivantes :

- température : 400°C
- pression totale : 2,5 MPa
- H2/HC= 8,5 mol/mol
- WHSV = 4$h^{-1}$ (masse de charge par gramme de catalyseur et par heure).

Les performances catalytiques du catalyseur C1 sont reportées dans le tableau 7.

Tableau 7 : Performances catalytiques du catalyseur C1 pour la dismutation du toluène.

| | |
|---|---|
| Conversion globale (%) | 49,7 |
| <u>Rendements (%poids)</u> | |
| Légers ($C_1$-$C_4$) | 2,5 |
| Benzène | 22,2 |
| xylènes | 21,1 |
| Ethylbenzène | 0,8 |
| Lourds | 3.1 |

Le catalyseur C1 est actif dans la mise en oeuvre du procédé de dismutation du toluène. Le benzène et le xylène sont produits avec un rendement satisfaisant.

## Revendications

1. Catalyseur comprenant au moins une zéolithe IZM-2 et au moins une matrice, ledit catalyseur comprenant de 4 à

60% poids d'au moins ladite zéolithe IZM-2 et de 40 à 96% poids d'au moins un matrice et ladite zéolithe présentant un diagramme de diffraction des rayons X incluant au moins les raies inscrites dans le tableau ci-dessous :

| 2 thêta (°) | $d_{hkl}$ (Å) | Irel | 2 thêta (°) | $d_{hkl}$ (Å) | Irel |
|---|---|---|---|---|---|
| 5,07 | 17,43 | ff | 19,01 | 4,66 | ff |
| 7,36 | 12,01 | FF | 19,52 | 4,54 | ff |
| 7,67 | 11,52 | FF | 21,29 | 4,17 | m |
| 8,78 | 10,07 | F | 22,44 | 3,96 | f |
| 10,02 | 8,82 | ff | 23,10 | 3,85 | mf |
| 12,13 | 7,29 | ff | 23,57 | 3,77 | f |
| 14,76 | 6,00 | ff | 24,65 | 3,61 | ff |
| 15,31 | 5,78 | ff | 26,78 | 3,33 | f |
| 15,62 | 5,67 | ff | 29,33 | 3,04 | ff |
| 16,03 | 5,52 | ff | 33,06 | 2,71 | ff |
| 17,60 | 5,03 | ff | 36,82 | 2,44 | ff |
| 18,22 | 4,87 | ff | 44,54 | 2,03 | ff |

où FF = très fort ; F = fort ; m = moyen ; mf = moyen faible ; f = faible ; ff = très faible et présentant une composition chimique, exprimée sur une base anhydre, en termes de moles d'oxydes, par la formule générale suivante : $XO_2$: $aY_2O_3$ : $bM_nO$ dans laquelle X représente au moins un élément tétravalent, Y représente au moins un élément trivalent et M est au moins un métal alcalin et/ou un métal alcalino-terreux, a et b représentant respectivement le nombre de moles de $Y_2O_3$ et $M_nO$ et a est compris entre 0,001 et 0,5, b est compris entre 0 et 1 et n est compris entre 1 et 2.

**2.** Catalyseur selon la revendication 1 tel que X est le silicium et Y est l'aluminium.

**3.** Catalyseur selon la revendication 1 ou la revendication 2 tel que ladite zéolithe se présente sous sa forme protonée.

**4.** Catalyseur selon la revendication 1 ou la revendication 2 tel que ladite zéolithe se présente sous sa forme basique.

**5.** Catalyseur selon l'une des revendications 1 à 4 tel quel ladite matrice contient de l'alumine.

**6.** Catalyseur selon l'une des revendications 1 à 5 tel qu'il comprend au moins un métal additionnel choisi parmi les métaux des groupes IIIB, IVA et IVB.

**7.** Procédé de production d'au moins un ester alcoolique à partir d'au moins un composé appartenant à la famille des triglycérides et d'au moins un composé portant une fonction alcool réalisé en présence d'au moins un catalyseur selon l'une des revendications 1 à 6.

**8.** Procédé selon la revendication 7 tel que ledit catalyseur comprend au moins une zéolithe IZM-2 soit sous forme hydrogène soit sous forme basique.

**9.** Procédé de transformation d'au moins un composé aliphatique ayant de 1 à 18 atomes de carbone et portant une fonction alcool, ledit procédé étant réalisé en présence d'au moins un catalyseur selon l'une des revendications 1 à 6.

**10.** Procédé selon la revendication 9 tel que ladite transformation mise en jeu est une réaction de déshydratation au cours de laquelle ledit composé aliphatique portant une fonction alcool est déshydraté en oléfine(s) avec production d'eau.

**11.** Procédé selon la revendication 9 tel que ladite transformation mise en jeu réalise simultanément dans un même réacteur la déshydratation dudit composé aliphatique en oléfine(s) et l'oligomérisation de(s)dite(s) oléfine(s).

**12.** Procédé d'oligomérisation d'une charge oléfinique consistant en la mise en contact de ladite charge avec au moins un catalyseur selon l'une des revendications 1 à 6.

**13.** Procédé de production de phénylalcanes comprenant une réaction d'alkylation d'au moins un composé aromatique par au moins une oléfine linéaire, ledit procédé étant réalisé en présence d'au moins un catalyseur selon l'une des revendications 1 à 6.

**14.** Procédé selon la revendication 13 tel que ledit composé aromatique est le benzène et ladite oléfine linéaire comprend de 9 à 16 atomes de carbone par molécule.

**15.** Procédé de dismutation du toluène pour produire du benzène et des xylènes réalisé en présence d'au moins un catalyseur selon l'une des revendications 1 à 6.

## Claims

**1.** A catalyst comprising at least one IZM-2 zeolite and at least one matrix, said catalyst compising from 4 to 60% by weight of at least said IZM-2 zeolite and from 40 to 96% by weight of at least one matrix, said zeolite having an X ray diffraction diagram including at least the peaks recorded in the table below.

| 2 theta (°) | $d_{hkl}$ (Å) | $I_{rel}$ | 2 theta (°) | $d_{hkl}$ (Å) | $I_{rel}$ |
|---|---|---|---|---|---|
| 507 | 17.43 | Vw | 19.01 | 4 66 | Vw |
| 7.36 | 12 01 | Vs | 19.52 | 4 54 | Vw |
| 767 | 11.52 | Vs | 21 29 | 4 17 | M |
| 878 | 10 07 | S | 22 44 | 3 96 | w |
| 10.02 | 8 82 | Vw | 23 10 | 3 85 | Mw |
| 12 13 | 7 29 | Vw | 23 57 | 3 77 | W |
| 14 76 | 6 00 | Vw | 24 65 | 3 61 | Vw |
| 15.31 | 5 78 | Vw | 26 78 | 3 33 | W |
| 15 62 | 5 67 | Vw | 29 33 | 3 04 | Vw |
| 16 03 | 5 52 | Vw | 33 06 | 2 71 | Vw |
| 17.60 | 5 03 | Vw | 36 82 | 2 44 | Vw |
| 18 22 | 4 87 | Vw | 44 54 | 2 03 | Vw |

in which Vs = very strong, S = strong, M = medium; Mw = medium weak, W = weak; Vw = very weak, and having a chemical composition expressed, as the anhydrous base in terms of moles of oxides, by the following general formula $XO_2 \cdot aY_2O_3 \, bM_nO$, in which X represents at least one tetravalent element, Y represents at least one trivalent element and M is at least one alkali metal and/or alkaline-earth metal, a and b respectively representing the number of moles of $Y_2O_3$ and $M_nO$, and a is in the range 0.001 to 0.5, b is in the range 0 to 1 and n is in the range 1 to 2

**2.** A catalyst according to claim 1, in which X is silicon and Y is aluminium,

**3.** A catalyst according to claim 1 or claim 2, in which said zeolite is in the protonated form.

**4.** A catalyst according to claim 1 or claim 2, in which said zeolite is in its basic form

**5.** A catalyst according to one of claims I to 4, m which said matrix contains alumina.

**6.** A catalyst according to one of claims 1 to 5, in which it comprises at least one additional metal selected from metals from groups IIIb, IVa and IVb.

**7.** A process for the production of at least one alcoholic ester from at least one compound belonging to the triglycerides family and at least one compound carrying an alcohol function carried out in the presence of at least one catalyst in accordance with one of claims 1 to 6.

**8.** A process according to claim 7, in which said catalyst comprises at least one IZM-2 zeolite in the hydrogen form or in the basic form.

**9.** A process for the transformation of at least one aliphatic compound containing I to 18 carbon atoms and carrying an alcohol function, said process being carried out in the presence of at least one catalyst according to one of claims I to 6.

**10.** A process according to claim 9, in which said transformation which takes place is a dehydration reaction during which said aliphatic compound carrying an alcohol function is dehydrated to olefin(s) with the production of water.

**11.** A process according to claim 9, in which said transformation which takes place simultaneously dehydrates said aliphatic compound to olefin(s) and oligomerizes said olefin(s) in the same reactor.

**12.** A process for oligomerizing an olefinic feed, consisting of bringing said feed into contact with at least one catalyst in accordance with one of claims 1 to 6.

**13.** A process for producing phenylalkanes, comprising a reaction for alkylation of at least one aromatic compound by at least one linear olefin, said process being carried out in the presence of at least one catalyst in accordance with one of claims 1 to 6.

**14.** A process according to claim 13, in which said aromatic compound is benzene and said linear olefin contains 9 to 16 carbon atoms per molecule.

**15.** A process for the disproportionation of toluene to produce benzene and xylenes carried out in the presence of at least one catalyst according to claims 1 to 6

**Patentansprüche**

**1.** Katalysator, umfassend mindestens einen IZM-2-Zeolithen und mindestens eine Matrix, wobei der Katalysator mindestens 4 bis 60 Gew.-% des IZM-2-Zeolithen und mindestens 40 bis 96 Gew.% einer Matrix umfasst, und der Zeolith ein Röntgendiffraktionsdiagramm aufweist, das mindestens die Linien enthält, die in der nachfolgenden Tabelle eingetragen sind:

| 2 theta (°) | $d_{hkl}$ (Å) | $I_{rel}$ | 2 theta (°) | $d_{hkl}$ (Å) | $I_{rel}$ |
|---|---|---|---|---|---|
| 5,07 | 17,43 | ff | 19,01 | 4,66 | ff |
| 7,36 | 12,01 | FF | 19,52 | 4,54 | ff |
| 7,67 | 11,52 | FF | 21,29 | 4,17 | m |
| 8,78 | 10,07 | F | 22,44 | 3,96 | f |
| 10,02 | 8,82 | ff | 23,10 | 3,85 | mf |
| 12,13 | 7,29 | ff | 23,57 | 3,77 | F |
| 14,76 | 6,00 | ff | 24,65 | 3,61 | ff |
| 15,31 | 5,78 | ff | 26,78 | 3,33 | f |
| 15,62 | 5,67 | ff | 29,33 | 3,04 | ff |
| 16,03 | 5,52 | ff | 33,06 | 2,71 | ff |
| 17,60 | 5,03 | ff | 36,82 | 2,44 | ff |
| 18,22 | 4,87 | ff | 44,54 | 2,03 | ff |

wobei FF = sehr stark ; F = stark; m = mittel; mf = mittel stark; f = schwach; ff = sehr schwach ist, und eine chemische Zusammensetzung aufweist, die auf wasserfreier Basis in Moloxid und durch die folgende allgemeine Formel ausgedrückt wird: $XO_2$ : $aY_2O_3$ : $bM_nO$, worin X für mindestens ein tetravalentes Element steht, Y für mindestens ein trivalentes Element steht und M mindestens ein alkalisches Metall und/oder ein erdalkalisches Metall ist, a und b jeweils für die Molanzahl von $Y_2O_3$ und $M_nO$ stehen und a im Bereich zwischen 0,001 und 0,5 liegt, b im Bereich zwischen 0 und 1 liegt und n im Bereich zwischen 1 und 2 liegt.

**2.** Katalysator nach Anspruch 1, wobei X Silicium ist und Y Aluminium ist.

**3.** Katalysator nach Anspruch 1 oder Anspruch 2, wobei der Zeolith in protonierter Form vorliegt.

**4.** Katalysator nach Anspruch 1 oder Anspruch 2, wobei der Zeolith in basischer Form vorliegt.

**5.** Katalysator nach einem der Ansprüche 1 bis 4, wobei die Matrix Aluminiumoxid enthält.

**6.** Katalysator nach einem der Ansprüche 1 bis 5, wobei er mindestens ein zusätzliches Metall umfasst, ausgewählt aus Metallen der Gruppen IIIB, IVA und IVB.

**7.** Verfahren zur Herstellung mindestens eines Alkoholesters aus mindestens einer Verbindung, die zur Familie der Triglyceride gehört, und mindestens einer Verbindung, die eine Alkoholfunktion trägt, das in Gegenwart von mindestens einem Katalysator nach einem der Ansprüche 1 bis 6 durchgeführt wird.

**8.** Verfahren nach Anspruch 7, wobei der Katalysator mindestens einen IZM-2-Zeolithen , entweder in Wasserstoffform oder in basischer Form umfasst.

**9.** Verfahren zur Umwandlung mindestens einer aliphatischen Verbindung, die 1 bis 18 Kohlenstoffatome aufweist und die eine Alkoholfunktion trägt, wobei das Verfahren in Gegenwart mindestens eines Katalysators nach einem der Ansprüche 1 bis 6 durchgeführt wird.

**10.** Verfahren nach Anspruch 9, wobei die Umwandlung, die eingesetzt wird, eine Dehydratationsreaktion ist, in deren Verlauf die aliphatische Verbindung, die eine Alkoholfunktion trägt, unter Wasserproduktion zu Olefin(en) dehydratisiert wird.

**11.** Verfahren nach Anspruch 9, wobei die Umwandlung, die eingesetzt wird, in ein und demselben Reaktor gleichzeitig die Dehydratation der aliphatischen Verbindung zu Olefin(en) und die Oligomerisation des (der) Olefins(e) durchführt.

**12.** Verfahren zur Oligomerisation einer Olefinbeschickung, das aus dem Inkontaktbringen der Beschickung mit mindestens einem Katalysator nach einem der Ansprüche 1 bis 6 besteht.

**13.** Verfahren zur Herstellung von Phenylalkanen, umfassend eine Alkylierungsreaktion mindestens einer aromatischen Verbindung durch mindestens ein lineares Olefin, wobei das Verfahren in Gegenwart mindestens eines Katalysators nach einem der Ansprüche 1 bis 6 durchgeführt wird.

**14.** Verfahren nach Anspruch 13, wobei die aromatische Verbindung Benzol ist und das lineare Olefin 9 bis 16 Kohlenstoffatome je Molekül umfasst.

**15.** Verfahren zur Toluoldismutation, um Benzol und Xylole herzustellen, das in Gegenwart mindestens eines Katalysators nach einem der Ansprüche 1 bis 6 durchgeführt wird.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 1953118 A